(19) Europäisches Patentamt
European Patent Office
Office européen des brevets

(11) **EP 1 144 006 B1**

(12) **EUROPEAN PATENT SPECIFICATION**

(45) Date of publication and mention
of the grant of the patent:
**17.10.2007 Bulletin 2007/42**

(21) Application number: **99966466.7**

(22) Date of filing: **16.12.1999**

(51) Int Cl.:
*A61K 39/21* (2006.01)    *A61K 39/40* (2006.01)
*A61K 39/42* (2006.01)    *C12N 5/06* (2006.01)
*C12N 7/00* (2006.01)    *C12N 7/04* (2006.01)
*C12N 7/06* (2006.01)    *C12Q 1/70* (2006.01)
*A61K 39/395* (2006.01)    *C07K 16/28* (2006.01)

(86) International application number:
**PCT/US1999/030345**

(87) International publication number:
**WO 2000/035409 (22.06.2000 Gazette 2000/25)**

(54) **CCR5 Antibody PA14**

CCR5 Antikörper PA14

Anticorps CCR5 PA14

(84) Designated Contracting States:
**AT BE CH CY DE DK ES FI FR GB GR IE IT LI LU MC NL PT SE**

(30) Priority: **16.12.1998 US 212793**
**16.12.1998 US 112532 P**

(43) Date of publication of application:
**17.10.2001 Bulletin 2001/42**

(60) Divisional application:
**07014859.8**

(73) Proprietor: **PROGENICS PHARMACEUTICALS, INC.**
**Tarrytown, NY 10591 (US)**

(72) Inventors:
• **OLSON, William, C.**
**Ossining, NY 10562 (US)**
• **MADDON, Paul, J.**
**Scarsdale, NY 10583 (US)**

(74) Representative: **Almond-Martin, Carol et al**
**ERNEST GUTMANN - YVES PLASSERAUD S.A.S.**
**88, Boulevard des Belges**
**69452 Lyon Cedex 06 (FR)**

(56) References cited:
**WO-A-97/45543      WO-A-97/47318**
**WO-A-98/18826**

• WU L ET AL: "CCR5 LEVELS AND EXPRESSION PATTERN CORRELATE WITH INFECTABILITY BY MACROPHAGE-TROPIC HIV-1, IN VITRO" JOURNAL OF EXPERIMENTAL MEDICINE, TOKYO, JP, vol. 185, no. 9, 5 May 1997 (1997-05-05), pages 1681-1691, XP002944844 ISSN: 0022-1007
• WU L ET AL: "INTERACTION OF CHEMOKINE RECEPTOR CCR5 WITH ITS LIGANDS: MULTIPLE DOMAINS FOR HIV-1 GP120 BINDING AND A SINGLE DOMAIN FOR CHEMOKINE BINDING" JOURNAL OF EXPERIMENTAL MEDICINE, TOKYO, JP, vol. 186, no. 8, October 1997 (1997-10), pages 1373-1381, XP002944588 ISSN: 0022-1007
• GHORPADE ANUJA ET AL: "Role of the beta-chemokine receptors CCR3 and CCR5 in human immunodeficiency virus type 1 infection of monocytes and microglia" JOURNAL OF VIROLOGY, vol. 72, no. 4, April 1998 (1998-04), pages 3351-3361, XP002296645 ISSN: 0022-538X
• SCHOLS D ET AL: "CD26-processed RANTES (3_68), but not intact RANTES, has potent anti-HIV activity" ANTIVIRAL RESEARCH, ELSEVIER SCIENCE BV., AMSTERDAM, NL, vol. 39, no. 3, October 1998 (1998-10), pages 175-187, XP002102983 ISSN: 0166-3542
• YLISASTIGUI LOYDA ET AL: "Synthetic full-length and truncated RANTES inhibit HIV-1 infection of primary macrophages" AIDS (LONDON), vol. 12, no. 9, 18 June 1998 (1998-06-18), pages 977-984, XP009036542 ISSN: 0269-9370

- MCKNIGHT A ET AL: "INHIBITION OF HUMAN IMMUNODEFICIENCY VIRUS FUSION BY A MONOCLONAL ANTIBODY TO A CORECEPTOR (CXCR4) IS BOTH CELL TYPE AND VIRUS STRAIN DEPENDENT" JOURNAL OF VIROLOGY, THE AMERICAN SOCIETY FOR MICROBIOLOGY, US, vol. 71, no. 2, February 1997 (1997-02), pages 1692-1696, XP002921360 ISSN: 0022-538X
- SIMMONS G ET AL: "POTENT INHIBITION OF HIV-1 INFECTIVITY IN MACROPHAGES AND LYMPHOCYTES BY A NOVEL CCR5 ANTOGONIST" SCIENCE, AMERICAN ASSOCIATION FOR THE ADVANCEMENT OF SCIENCE,, US, vol. 276, 1997, pages 276-279, XP000887293 ISSN: 0036-8075
- OLSON W C ET AL: "Differential inhibition of human immunodeficiency virus type 1 fusion, gp120 binding, and CC-chemokine activity by monoclonal antibodies to CCR5" JOURNAL OF VIROLOGY, THE AMERICAN SOCIETY FOR MICROBIOLOGY, US, vol. 73, no. 5, May 1999 (1999-05), pages 4145-4155, XP002290005 ISSN: 0022-538X
- TILLEY ET AL.: 'Potent Neutralization of HIV-1 by Human and Chimpanzee Monoclonal Antibodies Directed Against Three Distinct Epitope Clusters of gp120' SIXIEME COLLQUE DES CENT GARDES, 1991, pages 211 - 216, XP002927934
- TILLEY ET AL.: 'Synergistic Neutralization of HIV-1 by Human Monoclonal antibodies Against the V3 Loop and the CD4-Binding Site of gp120' AIDS RESEARCH AND HUMAN RETROVIRUSES, vol. 8, no. 4, 1992, pages 461 - 467, XP002045539
- ALLAWAY ET AL.: 'Synergistic Inhibition of HIV-1 Envelope-Mediated Cell fusion by CD4-Based Molecules in Combination with Antibodies to gp120 or gp41' AIDS RESEARCH AND HUMAN RETROVIRUSES, vol. 9, no. 7, 1993, pages 581 - 587, XP002927935
- CHOE ET AL.: 'The Beta-Chemokine Receptors CCR3 and CCR5 Facilitate Infection by Primary HIV-1 Isolates' CELL, vol. 85, no. 85, 28 June 1996, pages 1135 - 1148, XP002107164
- DORANZ ET AL.: 'A Dual-Tropic Primary HIV-1 Isolate That Uses Fusin and the Beta-Chemokine Receptors CKR-5, CKR-3 and CKR-2b as Fusion Cofactors' CELL, vol. 85, no. 7, 28 June 1996, pages 1149 - 1158, XP002028000
- DRAGIC ET AL.: 'HIV-1 Entry Into CD4+ Cells is Mediated by the Chemokine Receptor CC-CKR-5' NATURE, vol. 381, no. 6584, 20 June 1996, pages 667 - 673, XP002927936
- DENG ET AL.: 'Identification of a Major Co-Receptor for Primary Isolates of HIV-1' NATURE, vol. 381, 20 June 1996, pages 661 - 666, XP002028001
- FENG ET AL.: 'HIV-1 Entry Cofactor: Functional cDNA Cloning of a Seven-Transmembrane, G Protein-Coupled Receptor' SCIENCE, vol. 272, 10 May 1996, pages 872 - 877, XP002027999

**Description**

**Background of the Invention**

Human immunodeficiency virus type 1 (HIV-1) induces viral-to-cell membrane fusion to gain entry into target cells (8, 15, 66). The first high-affinity interaction between the virion and the cell surface is the binding of the viral surface glycoprotein gp120 to the CD4 antigen (13, 30, 41, 42). This in turn induces conformational changes in gp120, which enable it to interact with one of several chemokine receptors (4, 5, 21, 36). The CC-chemokine receptor CCR5 is the major co-receptor for macrophage-tropic (R5) strains, and plays a crucial role in the sexual transmission of HIV-1 (4, 5, 21, 36). T cell line-tropic (X4) viruses use CXCR4 to enter target cells, and usually, but not always, emerge late in disease progression or as a consequence of virus propagation in tissue culture (4, 5, 21, 36). Some primary HIV-1 isolates are dual-tropic (R5X4) since they can use both co-receptors, though not always with the same efficiency (11, 57). Mutagenesis studies coupled with the resolution of the gp120 core crystal structure demonstrated that the co-receptor binding site on gp120 comprises several conserved residues (32, 53, 65).

It has been demonstrated that tyrosines and negatively charged residues in the amino-terminal domain (Nt) of CCR5 are essential for gp120 binding to the co-receptor, and for HIV-1 fusion and entry (6, 18, 20, 22, 28, 31, 52, 54). Residues in the extracellular loops (ECL) 1-3 of CCR5 were dispensable for co-receptor function, yet the CCR5 interdomain configuration had to be maintained for optimal viral fusion and entry (24). This led to the conclusion either that gp120 forms interactions with a diffuse surface on the ECLs, or that the Nt is maintained in a functional conformation by bonds with residues in the ECLs. Studies with chimeric co-receptors and anti-CCR5 monoclonal antibodies have also shown the importance of the extracellular loops for viral entry (5, 54, 64).

Molecules that specifically bind to CCR5 and CXCR4 and block interactions with their ligands are a powerful tool to further probe the structure/function relationships of the co-receptors. Characterizing such compounds could also assist in designing effective therapeutic agents that target co-receptor-mediated steps of viral entry. Inhibitors of CCR5 or CXCR4 co-receptor function identified to date are diverse in nature and include small molecules, peptides, chemokines and their derivatives, and monoclonal antibodies (mAbs). The mechanisms of action of the small molecules that block entry by interfering with CXCR4 co-receptor function are not well understood (17, 49, 55, 68). One such inhibitor, the anionic small molecule AMD3100, depends on residues in ECL2 and the fourth trans-membrane (TM) domain of CXCR4 to inhibit viral entry, but it is not clear whether it does so by disrupting gp120 binding to CXCR4 or post-binding steps leading to membrane fusion (16, 34, 55). To date, no small molecules have been reported that specifically block CCR5-mediated HIV-1 entry. Inhibition of HIV-1 entry by chemokines is mediated by at least two distinct mechanisms: blockage of the gp120/co-receptor interaction and internalization of the chemokine/receptor complex (3, 26, 59, 63). The variant AOP-RANTES also inhibits recycling of CCR5 to the cell surface (40, 56). Variants such as RANTES 9-68 and Met-RANTES only prevent the gp120/CCR5 interaction and do not down-regulate CCR5 (67). SDF-1 variants presumably act through a similar mechanism to block viral entry mediated by CXCR4 (12, 27, 39). Only one anti-CXCR4 mAb, 12G5, has been characterized for its anti-viral properties. The efficiency of 12G5 inhibition of viral entry has been reported to be both cell-and isolate-dependent (43, 58). This mAb binds to the ECL2 of CXCR4, but the mechanism by which it inhibits entry is unknown (7). Few of the anti-CCR5 mAbs characterized to date efficiently prevent HIV-1 entry (28, 64). Interestingly, mAbs whose epitopes lie in the Nt domain of CCR5, which contains the gp120-binding site, inhibit viral fusion and entry less efficiently than mAb 2D7, whose epitope lies in ECL2. 2D7 also antagonizes CC-chemokine activity (64).

A panel of six murine mAbs, designated PA8, PA9, PA10, PA11, PA12 and PA14 have been isolated and characterized. All six mAbs specifically bound to CCR5+ cells but with different efficiencies that were cell type-dependent. Epitope mapping studies identified the residues that are important for mAb binding and also revealed information about the folding and interactions of the CCR5 extracellular domains. All mAbs inhibited HIV-1 fusion and entry, but there was no correlation between the ability of a mAb to inhibit fusion and entry and its ability to inhibit binding of gp120/sCD4 to CCR5+ cells.

This invention provides an anti-CCR5 chemokine receptor monoclonal antibody or fragment thereof as defined in the appendant claims as well as the use of said monoclonal antibody or fragment for the preparation of a composition for inhibiting HIV-1 fusion to, and viral entry into CD4+ cells.

**Brief Description of the Figures:**

Figure 1:

Binding of anti-CCR5 monoclonal antibodies to CCR5+ cells:

Flow cytometry was used to detect CCR5 protein expression on the surface of L1.2-CCR5+ cells and freshly isolated, PHA/IL-2-stimulated PBMC. Cells were incubated with saturating concentrations of each mAb, which were detected with a PE-labeled anti-mouse IgG reporter antibody. Results from a representative experiment are shown. Results for each mAb are expressed both in mean fluorescence intensities (m.f.i.) and in % gated cells. Since PA8-PA12 and PA14 are all of the IgG1 subclass, their m.f.i. are directly comparable. 2D7 is an IgG2a.

Figure 2:

CI values for different combinations of mAbs and viral inhibitors:

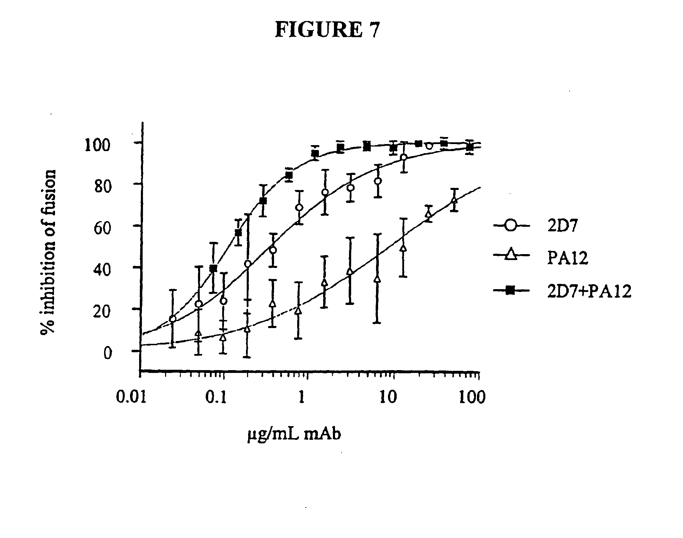

Experiments like those described in the legend of Fig. 7 were performed for different combinations of viral entry inhibitors. Anti-CCR5 mAbs were tested in combination with each other, CC-chemokines, and CD4-IgG2, which inhibits HIV-1 attachment to target cells. The PA11 and PA12 concentration range was 0-250 $\mu$g/ml; the 2D7 and PA14 concentration range was 0-25 $\mu$g/ml; the RANTES concentration range was 0-250 ng/ml; the CD4-IgG2 concentration range was 0-25 $\mu$g/ml. The concentrations of single-agents or their mixtures required to produce 50% and 90% inhibition of fusion or entry were quantitatively compared in a term known as the Combination Index (CI).

Figure 3

$IC_{50}$ values for inhibition of cell-cell fusion, viral entry and gp120/sCD4 binding by anti-CCR5 mAbs:

For comparative purposes we have summarized the $IC_{50}$ values obtained in the different assays that the anti-CCR5 mAbs were tested in. $IC_{50}$ values were only calculated for mAbs that could inhibit >90% of fusion, entry or binding.

Figure 4

Epitope mapping of anti-CCR5 mAbs:

A two color staining protocol was used to assess binding of mAbs to mutant CCR5 proteins, tagged at the C-terminus with the HA peptide. HeLa cells expressing CCR5 point mutants were incubated with saturating concentrations of each mAb followed by detection with a PE-labeled anti-mouse IgG. Cell surface co-receptor expression was measured by double-staining of the cells with a FITC labeled anti-HA mAb. The four grids correspond to the four extracellular domains of CCR5. The first row of every grid indicates the amino acid sequence of the corresponding CCR5 extracellular domain (SEQ ID NOs. 1-4). Binding of anti-CCR5 mAbs to the alanine mutant of each residue is expressed as a percentage of binding to wild-type CCR5, as described in Materials and Methods.

Figure 5

Inhibition of calcium mobilization into CCR5+ cells by anti-CCR5 mAbs:

L1.2-CCR5+ cells were loaded with Indo-1AM and stimulated sequentially with an anti-CCR5 mAb or PBS, followed with RANTES (a). Fluorescence changes were measured with a spectrofluorometer and the tracings are from a representative experiment. Calcium flux inhibition by PA14 and 2D7 was tested for a wide range of mAb concentrations (b). Results are plotted as % inhibition of calcium influx = [1- (relative fluorescence in the presence of mAb $\div$ relative fluorescence in the absence of mAb)] x 100%, and are means of values from three independent experiments.

Figure 6

Inhibition of CCR5 co-receptor function by anti-CCR5 mAbs:

Inhibition of cell-cell fusion by anti-CCR5 mAbs was tested in the RET assay (a). 0-250$\mu$g/ml of PA8-PA12, or 0-25$\mu$g/ml of PA14 or 2D7, were added to a mix of HeLa-EnV$_{JR-FL}$ and PM1 cells, labeled with F18 and R18 respectively. Fluorescence RET was measured after 4h of incubation. Results are mean values from three independent experiments and are expressed as % inhibition of fusion = [1-(% RET in the presence of mAb $\div$ % RET in the absence of mAb)] x 100%. Inhibition of HIV-1 entry by anti-CCR5 mAbs was tested in a single round of replication luciferase based entry assay (b). U87-CD4+CCR5+ cells were infected with NLluc+env- reporter virus carrying the JR-FL envelope in the presence of 0-250$\mu$g/ml of PA8-PA12, or 0-25$\mu$g/ml PA14 or 2D7. Luciferase activity (relative light units, r.l.u.) was measured in cell lysates 72h post-infection. Results are from a representative experiment and are expressed as % inhibition of entry = [1-(r.l.u. in the presence of mAb $\div$ r.l.u. in the absence of mAb)] x 100%. Binding of biotinylated [b] gp120, sCD4 and b-gp120-CD4 complexes to L1.2-CCR5$^+$ cells (c). Strong binding is observed when gp120 derived from the R5 virus HIV-1$_{JR-FL}$ is complexed with an equimolar amount of sCD4. No binding is observed in the absence of sCD4 or for gp120 derived from the X4 virus HIV-1$_{LAI}$. Background binding to CCR5- L1.2 cells has been subtracted from all curves. Inhibition of gp120/sCD4 binding to L1.2-CCR5$^+$ cells was tested in the presence of varying concentrations of each antibody (d). Cells were preincubated in 96-well plates with an anti-CCR5 mAb followed by an incubation with a saturating concentration of biotinylated gp120/sCD4. Finally, binding of PE-labeled streptavidin to cells was measured using a fluorescence plate reader. Results are from a representative experiment and are expressed as % inhibition of gp120/sCD4 binding = [1-(m.f.i. in the presence of mAb ÷ m.f.i. in the absence of mAb)] x 100%.

Figure 7:

Synergistic inhibition of cell-cell fusion by PA12 and 2D7:

Dose-response curves were obtained for the mAbs used individually and in combination. 0-50μg/ml of PA12, 0-25μg/ml 2D7, or a combination of the two in a 2:1 ratio, were added to a mix of HeLa-Env$_{JR-FL}^+$ and PM1 cells, labeled with R18 and F18 respectively. Fluorescence RET was measured after 4 hours of incubation. Results are expressed as % inhibition of fusion and are the means of values from three independent experiments. Data were analyzed using the median effect principle, which can be written

$$f \quad = \quad 1/[1 \ + \ (K/c)^m] \qquad\qquad (1)$$

where f is the fraction affected/inhibited, c is concentration, K is the concentration of agent required to produce the median effect, and m is an empirical coefficient describing the shape of the dose-response curve. Equation (1) is a generalized form of the equations describing Michaelis-Menton enzyme kinetics, Langmuir adsorption isotherms, and Henderson-Hasselbalch ionization equilibria, for which m = 1. In the present case, K is equal to the IC$_{50}$ value. K and m were determined by curve-fitting the dose-response curves and Equation (1) was rearranged to allow calculation of c for a given f. The best-fit parameters for K and c are 8.8μg/ml and 0.54 for PA12, 0.36μg/ml and 0.68 for 2D7, and 0.11μg/ml and 1.1 for their combination. These curves are plotted and indicate a reasonable goodness-of-fit between experiment and theory.

**Detailed Description of the invention:**

This invention provides an anti-CCR5 chemokine receptor monoclonal antibody or a fragment thereof as defined in the claims.

The disclosure also relates to a composition for inhibiting HIV-1 infection comprising at least two compounds in synergistically effective amounts for inhibiting HIV-1 infection, wherein at least one of the compounds prevents with the productive interaction between HIV-1 and an HIV-1 fusion co-receptor.

As used herein, "composition" means a mixture. The compositions include but are not limited to those suitable for oral, rectal, intravaginal, topical, nasal, opthalmic, or parenteral administration to a subject. As used herein, "parenteral" includes but is not limited to subcutaneous, intravenous, intramuscular, or intrasternal injections or infusion techniques.

As used herein, "HIV-1" means the human immunodeficiency virus type-1. HIV-1 includes but is not limited to extracellular virus particles and the forms of HIV-1 found in HIV-1 infected cells.

As used herein, "HIV-1 infection" means the introduction of HIV-1 genetic information into a target cell, such as by fusion of the target cell membrane with HIV-1 or an HIV-1 envelope glycoprotein$^+$ cell. The target cell may be a bodily cell of a subject. In the preferred embodiment, the target cell is a bodily cell from a human subject.

As used herein, "inhibiting HIV-1 infection" means the reduction of the amount of HIV-1 genetic information introduced into a target cell population as compared to the amount that would be introduced without said composition.

As used herein, "compound" means a molecular entity, including but not limited to peptides, polypeptides, and other organic or inorganic molecules and combinations thereof.

As used herein, "synergistically effective" means that the combined effect of the compounds when used in combination is greater than their additive effects when used individually.

As used herein, "productive interaction" means that the interaction of HIV-1 and the HIV-1 co-receptor would lead to the fusion of said HIV-1 or HIV-1 envelope glycoprotein$^+$ cell and the membrane bearing the co-receptor.

As used herein, "prevents the productive interaction" means that the amount of interaction is reduced as compared to the amount that would occur without the compound. The interactions may be prevented by masking or altering interactive regions on the co-receptor or HIV-1 or by altering the expression, aggregation, conformation, or association state of the co-receptor.

As used herein, "HIV-1 fusion co-receptor" means a cellular receptor that mediates fusion between the target cell expressing the receptor and HIV-1 or an HIV-1 envelope glycoprotein cell. HIV-1 fusion co-receptors include but are not limited to CCR5, CXCR4 and other chemokine receptors.

This invention also provides a composition which inhibits fusion of HIV-1 or an HIV-1 envelope glycoprotein$^+$ cell to a target cell, comprising at least two compounds in synergistically effective amounts for inhibiting fusion of HIV-1 or an HIV-1 envelope glycoprotein$^+$ cell to a target cell, wherein at least one of the compounds prevents the productive interaction between HIV-1 and an HIV-1 fusion co-receptor.

As used herein, "fusion" means the joining or union of the lipid bilayer membranes found on mammalian cells or viruses such as HIV-1. This process is distinguished from the attachment of HIV-1 to a target cell. Attachment is mediated by the binding of the HIV-1 exterior glycoprotein to the human CD4 receptor, which is not a fusion co-receptor.

As used herein, "inhibits" means that the amount is reduced as compared with the amount that would occur without the composition.

As used herein, "target cell" means a cell capable of being infected by or fusing with HIV-1 or HIV-1 infected cells.

As used herein, "chemokine" means a cytokine that can stimulate leukocyte movement. They may be characterized as either cys-cys or cys-X-cys depending on whether the two amino terminal cysteine residues are immediately adjacent or separated by one amino acid. It includes but is not limited to RANTES, MIP-1α, MIP-1β, SDF-1 or another chemokine which blocks HIV-1 infection.

In one embodiment of the above compositions, the co-receptor is a chemokine receptor. In the preferred embodiment of the above compositions, the chemokine receptor is CCR5 or CXCR4. Several other chemokine and related receptors are known to function as HIV co-receptors including but not limited to CCR2, CCR3, CCR8, STRL33, GPR-15, CX3CR1 and APJ (69).

As used herein, "chemokine receptor" means a member of a homologous family of seven-transmembrane spanning cell surface proteins that bind chemokines.

As used herein, "CCR5" is a chemokine receptor which binds members of the C-C group of chemokines and whose amino acid sequence comprises that provided in Genbank Accession Number 1705896 and related polymorphic variants.

As used herein, "CXCR4" is a chemokine receptor which binds members of the C-X-C group of chemokines and whose amino acid sequence comprises that provided in Genbank Accession Number 400654 and related polymorphic variants.

In one embodiment of the above compositions, at least one of the compounds is a nonpeptidyl molecule. In one embodiment, the nonpeptidyl molecule is the bicyclam compound AMD3100. (16).

As used herein, "nonpeptidyl molecule" means a molecule that does not consist in its entirety of a linear sequence of amino acids linked by peptide bonds. A nonpeptidyl molecule may, however, contain one or more peptide bonds.

In a preferred embodiment, the antibody is an anti-CCR5 antibody. The anti-CCR5 antibody includes but is not limited to PA14

Murine hybridomas secreting monoclonal antibodies PA8, PA9, PA10, PA11, PA12 and PA14, respectively, were deposited pursuant to and in satisfaction of, the requirements of the Budapest Treaty on the International Recognition of the Deposit of Microorganisms for the Purposes of Patent Procedure with the American Type Culture Collection (ATCC), 10801 University Boulevard, Manassas, Virginia 20110-2209 on December 2, 1998 under the following Accession Nos.: ATCC Accession No. HB-12605 (PA8), ATCC Accession No. HB-12606 (PA9), ATCC Accession No.12607 (PA10), ATCC Accession No. HB-12608 (P11), ATCC Accession No. HB-12609 (PA12), and ATCC Accession No. HB-126010 (PA14).

In another aspect the disclosure refers to a compositions, for inhibiting HIV-1 infection comprising two or more antibodies. The antibodies include but are not limited to PA8, PA9, PA10, PA11, PA12, PA14 and 2D7. In this composition the antibodies are in an appropriate ratio. The ratio ranges from 1:1 to 50:1.

As used herein, "antibody" means an immunoglobulin molecule comprising two heavy chains and two light chains and which recognizes an antigen. The immunoglobulin molecule may derive from any of the commonly known classes, including but not limited to IgA, secretory IgA, IgG and IgM. IgG subclasses are also well known to those in the art and include but are not limited to human IgG1, IgG2, IgG3 and IgG4. It includes, by way of example, both naturally occurring and non-naturally occurring antibodies. Specifically, "antibody" includes polyclonal and monoclonal antibodies, and monovalent and divalent fragments thereof. Furthermore, "antibody" includes chimeric antibodies, wholly synthetic antibodies, single chain antibodies, and fragments thereof. Optionally, an antibody can be labeled with a detectable marker. Detectable markers include, for example, radioactive or fluorescent markers. The antibody may be a human or nonhuman antibody. The nonhuman antibody may be humanized by recombinant methods to reduce its immunogenicity in man. Methods for humanizing antibodies are known to those skilled in the art.

As used herein, "monoclonal antibody," also designated as mAb, is used to describe antibody molecules whose primary sequences are essentially identical and which exhibit the same antigenic specificity. Monoclonal antibodies may be produced by hybridoma, recombinant, transgenic or other techniques known to one skilled in the art.

As used herein, "anti-chemokine receptor antibody" means an antibody which recognizes and binds to an epitope on a chemokine receptor. As used herein, "anti-CCR5 antibody" means a monoclonal antibody which recognizes and binds to an epitope on the CCR5 chemokine receptor.

As used herein, "appropriate ratio" means mass or molar ratios wherein the compounds are synergistically effective.

In one embodiment of the above compositions, at least one compound is a chemokine or chemokine derivative. The chemokines include but are not limited to RANTES, MIP-1α, MIP-1β, SDF-1 or a combination thereof. In this composition, the compounds are in an appropriate ratio. The chemokine derivatives include but are not limited to Met-RANTES, AOP-RANTES, RANTES 9-68, or a combination thereof.

As used herein, "chemokine derivative" means a chemically modified chemokine. The chemical modifications include but are not limited to amino acid substitutions, additions or deletions, non-peptidyl additions or oxidations. One skilled in the art will be able to make such derivatives.

In another embodiment of the above compositions, at least one compound is an antibody and at least one compound is a chemokine or chemokine derivative. In this composition, the compounds are in an appropriate ratio. The ratio ranges from 100:1 to 1000:1.

In another embodiment of the above compositions, at least one compound binds to the gp41 subunit of the HIV-1 envelope glycoprotein. In one embodiment, at least one compound is the T-20 peptide inhibitor of HIV-1 entry (70).

In another embodiment of the above compositions, at least one of the compounds inhibits the attachment of HIV-1 to a target cell. In one embodiment, at least one compound binds CD4. In one embodiment, at least one compound is an HIV-1 envelope glycoprotein. In one embodiment, at least one compound is an anti-CD4 antibody. In one embodiment, at least one compound binds to the HIV-1 envelope glyoprotein. In one embodiment, at least one compound is an antibody to the HIV-1 envelope glycoprotein. In one embodiment, at least one compound is a CD4-based protein. In one embodiment, at least one compound is CD4-IgG2.

In another embodiment of the above compositions, at least one compound is an antibody and at least one compound binds to an HIV-1 envelope glycoprotein. In one embodiment, the compound is a CD4-based protein. In one embodiment, the compound is CD4-IgG2. In this composition, the compounds are in an appropriate ratio. The ratio ranges from 1:1 to 10:1.

As used herein, "attachment" means the process that is mediated by the binding of the HIV-1 envelope glycoprotein to the human CD4 receptor, which is not a fusion co-receptor.

As used herein, "CD4" means the mature, native, membrane-bound CD4 protein comprising a cytoplasmic domain, a hydrophobic transmembrane domain, and an extracellular domain which binds to the HIV-1 gp120 envelope glycoprotein.

As used herein, "HIV-1 envelope glycoprotein" means the HIV-1 encoded protein which comprises the gp120 surface protein, the gp41 transmembrane protein and oligomers and precursors thereof.

As used herein, "CD4-based protein" means any protein comprising at least one sequence of amino acid residues corresponding to that portion of CD4 which is required for CD4 to form a complex with the HIV-1 gp120 envelope glycoprotein.

As used herein, "CD4-IgG2" means a heterotetrameric CD4-human IgG2 fusion protein encoded by the expression vectors deposited under ATCC Accession Numbers 75193 and 75194.

In one embodiment of the above compositions at least one of the compounds comprises a polypeptide which binds to a CCR5 epitope. In one embodiment, the epitope is located in the N-terminus, one of the three extracellular loop regions or a combination thereof. In one embodiment, the epitope is located in the N-terminus. The epitope can comprise N13 and Y15 in the N-terminus. The epitope can comprise Q4 in the N-terminus. In another embodiment, the epitope includes residues in the N-terminus and second extracellular loop. The epitope can comprise D2, Y3, Q4, S7, P8 and N13 in the N-terminus and Y176 and T177 in the second extracellular loop. The epitope can comprise D2, Y3, Q4, P8 and N13 in the N-terminus and Y176 and T177 in the second extracellular loop. The epitope can comprise D2 in the N-terminus and R168 and Y176 in the second extracellular loop. In one embodiment, the epitope is located in the second extra cellular loop. The epitope can comprise Q170 and K171 in the second extracellular loop. The epitope can comprise Q170 and E172 in the second extra cellular loop.

As used herein, the following standard abbreviations are used throughout the specification to indicate specific amino acids:

A=ala=alanine          R=arg=arginine
N=asn=asparagine       D=asp=aspartic acid
C=cys=cysteine         Q=gln=glutamine

(continued)

| | |
|---|---|
| E=glu=glutamic acid | G=gly=glycine |
| H=his=histidine | I=ile=isoleucine |
| L=leu=leucine | K=lys=lysine |
| M=met=methionine | F=phe=phenylalanine |
| P=pro=proline | S=ser=serine |
| T=thr=threonine | W=trp=tryptophan |
| Y=tyr=tyrosine | V=val=valine |

As used herein, "polypeptide" means two or more amino acids linked by a peptide bond.

As used herein, "epitope" means a portion of a molecule or molecules that forms a surface for binding antibodies or other compounds. The epitope may comprise contiguous or noncontiguous amino acids, carbohydrate or other nonpeptidyl moities or oligomer-specific surfaces.

As used herein, "N-terminus" means the sequence of amino acids spanning the initiating methionine and the first transmembrane region.

As used herein, "second extra cellular loop" means the sequence of amino acids that span the fourth and fifth transmembrane regions and are presented on the surface.

In one embodiment of the above compositions at least one of the compounds comprises a light chain of an antibody. In another embodiment of the above compositions at least one of the compounds comprises a heavy chain of an antibody. In another embodiment of the above compositions at least one of the compounds comprises the Fab portion of an antibody. In another embodiment of the above compositions at least one of the compounds comprises the variable domain of an antibody. In another embodiment, the antibody is produced as a single polypeptide or "single chain" antibody which comprises the heavy and light chain variable domains genetically linked via an intervening sequence of amino acids. In another embodiment of the above compositions at least one of the compounds comprises one or more CDR portions of an antibody.

As used herein, "heavy chain" means the larger polypeptide of an antibody molecule composed of one variable domain (VH) and three or four constant domains (CH1, CH2, CH3, and CH4), or fragments thereof.

As used herein, "light chain" means the smaller polypeptide of an antibody molecule composed of one variable domain (VL) and one constant domain (CL), or fragments thereof.

As used herein, "Fab" means a monovalent antigen binding fragment of an immunoglobulin that consists of one light chain and part of a heavy chain. It can be obtained by brief papain digestion or by recombinant methods.

As used herein, "F(ab')2 fragment" means a bivalent antigen binding fragment of an immunoglobulin that consists of both light chains and part of both heavy chains. It cen be obtained by brief pepsin digestion or recombinant methods.

As used herein, "CDR" or "complementarity determining region" means a highly variable sequence of amino acids in the variable domain of an antibody.

This disclosure relates to the above compositions and a pharmaceutically acceptable carrier. Pharmaceutically acceptable carriers are well known to those skilled in the art. Such pharmaceutically acceptable carriers may include but are not limited to aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers such as those based on Ringer's dextrose, and the like. Preservatives and other additives may also be present, such as, for example, antimicrobials, antioxidants, chelating agents, inert gases and the like.

This disclosure relates to a method of treating a subject afflicted with HIV-1 which comprises administering to the subject an effective dose of the above compositions.

As used herein, "subject" means any animal or artificially modified animal capable of becoming HIV-infected. Artificially modified animals include, but are not limited to, SCID mice with human immune systems. The animals include but are not limited to mice, rats, dogs, guinea pigs, ferrets, rabbits, and primates. In the preferred embodiment, the subject is a human.

As used herein, "treating" means either slowing, stopping or reversing the progression of an HIV-1 disorder. In the preferred embodiment, "treating" means reversing the progression to the point of eliminating the disorder. As used herein, "treating" also means the reduction of the number of viral infections, reduction of the number of infectious viral particles, reduction of the number of virally infected cells, or the amelioration of symptoms associated with HIV-1.

As used herein, "afflicted with HIV-1" means that the subject has at least one cell which has been infected by HIV-1.

As used herein, "administering" may be effected or performed using any of the methods known to one skilled in the art. The methods may comprise intravenous, intramuscular or subcutaneous means.

The dose of the composition of the invention will vary depending on the subject and upon the particular route of administration used. Dosages can range from 0.1 to 100,000 $\mu$g/kg. Based upon the composition, the dose can be delivered continuously, such as by continuous pump, or at periodic intervals. For example, on one or more separate occasions. Desired time intervals of multiple doses of a particular composition can be determined without undue experimentation by one skilled in the art.

As used herein, "effective dose" means an amount in sufficient quantities to either treat the subject or prevent the subject from becoming HIV-1 infected. A person of ordinary skill in the art can perform simple titration experiments to determine what amount is required to treat the subject.

This disclosure relates to a method of preventing a subject from contracting HIV-1 which comprises administering to the subject an effective dose of the above compositions.

As used herein, "contracting HIV-1" means becoming infected with HIV-1, whose genetic information replicates in and/or incorporates into the host cells.

This invention provides an anti-CCR5 monoclonal antibody. The antibody includes but is not limited to an antibody produced by one of the following hybridomas: PA8 (ATCC Accession No. HB-12605), PA9 (ATCC Accession No. HB-12606), PA10 (ATCC Accession No. 12607), PA11 (ATCC Accession No. HB-12608), PA12 (ATCC Accession No. HB-12609), and PA14 (ATCC Accession No. HB-12610).

This invention provides humanized forms of the above antibodies.

As used herein, "humanized" describes antibodies wherein some, most or all of the amino acids outside the CDR regions are replaced with corresponding amino acids derived from human immunoglobulin molecules. In one embodiment of the humanized forms of the antibodies, some, most or all of the amino acids outside the CDR regions have been replaced with amino acids from human immunoglobulin molecules but where some, most or all amino acids within one or more CDR regions are unchanged. Small additions, deletions, insertions, substitutions or modifications of amino acids are permissible as long as they would not abrogate the ability of the antibody to bind a given antigen. Suitable human immunoglobulin molecules would include IgG1, IgG2, IgG3, IgG4, IgA and IgM molecules. A "humanized" antibody would retain a similar antigenic specificity as the original antibody, i.e., in the present invention, the ability to bind CCR5.

This disclosure relates to isolated nucleic acid molecules encoding these anti-CCR5 monoclonal antibodies or their humanized versions. The nucleic acid molecule can be RNA, DNA or cDNA. In one embodiment, the nucleic acid molecule encodes the light chain. In one embodiment, the nucleic acid molecule encodes the heavy chain. In one embodiment, the nucleic acid encodes both the heavy and light chains. In one embodiment, one or more nucleic acid molecules encode the Fab portion. In one embodiment, one or more nucleic acid molecules encode CDR portions. In one embodiment, the nucleic acid molecule encodes the variable domain.

This invention will be better understood from the Experimental Details which follow. However, one skilled in the art will readily appreciate that the specific methods and results discussed are merely illustrative of the invention as described more fully in the claims which follow thereafter.

## Experimental Details:

### A. Materials and Methods

#### 1) Reagents

MAb 2D7 was purchased from Pharmingen (San Diego, CA) and CC-and CXC-chemokines were obtained from R&D Systems (Minneapolis, MN). CD4-IgG2 (1), soluble (s) CD4 (2) and recombinant HIV-1$_{JR-FL}$ gp120, were produced by Progenies Pharmaceuticals, Inc. (59).

#### 2) Isolation and purification of anti-CCR5 mAbs

L1.2-CCR5$^+$ cells (63) were incubated for 16h in the presence of 5mM sodium butyrate, which activates transcription from the cytomegalovirus (CMV) promoter that controls CCR5 expression, resulting in a 10-fold increase in cell surface co-receptor density. Female Balb/c mice were immunized intraperitoneally with $10^7$ L1.2-CCR5$^+$ cells at 3-week intervals, and administered an intravenous boost of $10^7$L1.2-CCR5$^+$ cells three days prior to splenectomy. Splenocytes were fused with the Sp2/0 cell line. In a primary screen, supernatants from ten thousand hybridoma cultures were tested; one hundred and twenty of these inhibited HIV-1 envelope-mediated fusion between PM1 cells (10), which naturally express CCR5 and CD4, and HeLa-Env$_{JR-FL}$$^+$ cells in a resonance energy transfer (RET) assay, as previously described (19, 38). Hybridomas that produced the most potently inhibitory supernatants and that also stained CCR5$^+$ cells were sub-cloned by limiting dilution. Ascites fluids were prepared by Harlan Bioproducts for Science, Inc. (Indianapolis, IN) from Balb/c mice that were injected with hybridomas producing the anti-CCR5 mAbs PA8, PA9, PA10, PA11, PA12 and PA14. The mAbs were individually purified to >95% homogeneity by precipitation with ammonium sulfate followed by protein-A chromatography. All mAbs were resuspended in phosphate buffered saline (PBS) at a final concentration of 5mg/ml.

3) Fluorescence activated cell sorting (FACS) analysis and epitope mapping of anti-CCR5 mAbs

Flow cytometry was used to detect cell-surface reactivity of mAbs PA8-PA12 and PA14 with CCR5. Sodium butyrate treated L1.2-CCR5$^+$ cells (10$^6$) were incubated with 0.25μg of antibody, for 20min at 4˚C in 0.1% sodium azide (NaN$_3$) in 50 μl of Dulbecco's PBS (DPBS). The CCR5 mAb 2D7 was used as a positive control, a non-specific murine IgG1 was used as a negative control. The cells were spun down, washed and incubated with phycoerythrin (PE)-labeled goat anti-mouse IgG (Caltag, Burlingame, CA) diluted 1:100, under the same conditions as the first antibody incubation. Finally, cells were analyzed by flow cytometry. PBMC were isolated and stimulated as previously described (60) and stained using similar methods.

A similar procedure was used for epitope mapping of the anti-CCR5 mAbs. A panel of seventy CCR5 point mutants has been described (20, 24, 52). The coding sequences of these proteins are sub-cloned into the pcDNA3.1 vector (Stratagene) from which transcription can be driven by a 5' T7-polymerase promoter. The CCR5 mutants carry a 9-residue hemaglutinin (HA) tag at the C-terminus for detection of protein in cell lysates or by flow cytometry. HeLa cells (2x10$^6$) were incubated for 5h with 20μg/ml lipofectin and an equal amount of wild-type or mutant CCR5-expressing plasmid in OPTI-MEM (Life Technologies, Gaithersburg, MD). The cells were then infected for 12h with 2x10$^7$ p.f.u. of vTF7 (23) to boost CCR5 expression, detached with 2mM ethylenediamine tetracetic acid (EDTA) in PBS and washed once with binding buffer (1% BSA, 0.05% NaN$_3$ in DPBS). Cells (1x10$^6$) were surface labeled with mAbs as described in the previous paragraph, washed once with the incubation buffer and resuspended in 1ml of 1x FACSlyse in water (Becton Dickinson) for 30min at room temperature, to permeabilize the cell membranes. The cells were then spun down, washed with the incubation buffer and incubated for 1h at 37˚C with 4μg/ml of a fluorescein isothiocyanate (FITC)-labeled mouse anti-HA mAb (BabCo, Richmond, CA) for intracellular labeling. Finally, cells were washed once with binding buffer and once with DPBS, resuspended in 1% formaldehyde in PBS and analyzed by flow cytometry. The extent of binding of a mAb to mutant CCR5 was determined by the equation (mutant CCR5 PE m.f.i. / wt CCR5 PE m.f.i.) / (mutant CCR5 FITC m.f.i. / wt CCR5 FITC m.f.i.) x100%. This normalizes mAb binding for mutant co-receptor expression levels.

4) gp120/sCD4-binding assay

gp120 was biotinylated using NHS-biotin (Pierce, Rockford, IL) according to the manufacturer's instructions, and uncoupled biotin was removed by diafiltration. Sodium butyrate-treated L1.2-CCR5$^+$ cells were incubated with varying dilutions of an equimolar mixture of sCD4 and biotinylated gp120, or 1.25μg/ml of sCD4 and 2.5μg/ml of biotinylated gp120 in the presence of varying concentrations of anti-CCR5 mAbs PA8-PA12, PA14, 2D7 or a non-specific murine IgG1, for 1h at room temperature in 0.1% NaN$_3$ in DPBS. Cells were washed with the incubation buffer and incubated with streptavidin-PE (Becton Dickinson) diluted 1:50, for 1h at room temperature. Finally, cells were washed with binding buffer and analyzed using a fluorescence plate reader (Perspective Biosystems, Framingham, MA).

5) Inhibition of envelope-mediated cell-cell fusion and HIV-1 entry by anti-CCR5 mAbs

HIV-1 envelope-mediated fusion between HeLa-Env$_{JR-FL}$$^+$ and PM1 cells was detected using the RET assay. Equal numbers (2x10$^4$) of fluorescein octadecyl ester (F18)-labeled envelope-expressing cells and octadecyl rhodamine (R18)-labeled PM1 cells were plated in 96-well plates in 15% fetal calf serum in DPBS and incubated for 4h at 37˚C in the presence of varying concentrations of the anti-CCR5 mAbs, PA8-PA12, PA14, 2D7 or a non-specific murine IgG1. Fluorescence RET was measured with a Cytofluor plate-reader (PerSeptive Biosystems) and % RET was determined as previously described (38). NLluc$^+$env$^-$ viruses complemented in *trans* by envelope glycoproteins from JR-FL or Gun-1 were produced as previously described (20). U87MG-CD4$^+$CCR5$^+$ cells (14) were infected with chimeric, reporter viruses containing 50-100ng/ml p24 in the presence of varying concentrations of the individual mAbs. After 2h at 37˚C, virus-containing media were replaced by fresh, mAb-containing media. Fresh media, without antibodies, were added again after 12 hours. After a total of 72h, 100μl of lysis buffer (Promega) were added to the cells and luciferase activity (r.l.u.) was measured as described (20). The % inhibition of HIV-1 infection is defined as [1-(r.l.u in the presence of antibody / r.l.u in the absence of antibody)] x 100%.

6) Calcium signaling assays

The fluorochrome Indo-1AM (Molecular Probes, Eugene, OR) was added to sodium butyrate treated L1.2-CCR5$^+$ cells at a final concentration of 5μM. After incubation at 37°C for 30min, the cells were washed once and resuspended in Hank's buffered saline. Cells (10$^6$) were stimulated sequentially with an anti-CCR5 mAb or PBS, followed 60s later with RANTES. MAbs PA8-PA12 and PA14 were used at a concentration of 100μg/ml, 2D7 at 20μg/ml and RANTES at 250ng/ml. Calcium flux inhibition by PA14 and 2D7 was also tested for a wide range of mAb concentrations, ranging from 0-100μg/ml. Intracellular calcium levels were monitored using a Perkin-Elmer LS-50S fluorescence spectrophotometer by measuring the ratio of fluorescence emissions at 402nm (bound dye) and 486nm (free dye) following excitation at 358nm.

B. **Results and Discussion**

1) Isolating anti-CCR5 monoclonal antibodies PA8, PA9, PA10, PA11, PA12 and PA14

It was found that peptides corresponding to the extracellular domains of CCR5 are inefficient at raising specific, high-titer antibody responses against the native, cell surface receptor (50). Balb/C mice were immunized, therefore, with L1.2-CCR5$^+$ cells and hybridoma culture supernatants were tested for their ability to inhibit JR-FL envelope-mediated membrane fusion with CD4$^+$CCR5$^+$ PM1 cells in the RET assay (19, 38). Even though well over a hundred supernatants inhibited cell-cell fusion by >50%, only six - designated PA8, PA9, PA10, PA11, PA12 and PA14 - specifically and intensely stained L1.2-CCR5$^+$ but not the parental L1.2 cells, as demonstrated by flow cytometry (data not shown). Based on previous experience, it was assumed that the other mAbs capable of inhibiting cell-cell fusion were probably directed against cell surface adhesion molecules such as LFA-1 (37). Hybridomas PA8-PA12 and PA14 were determined by isotyping ELISA (Cappell, Durham, NC) to secrete IgG1 mAbs. Ascites fluids were prepared from Balb/C mice that were injected with the six hybridomas and the IgG1 fractions were purified. PA8, PA9, PA11, PA12 and PA14 exhibited distinct isoelectric focussing profiles, whereas PA10 had a very similar profile to that of PA9 and therefore may be a second isolate of the same mAb (data not shown).

2) MAb binding to CCR5+ cells

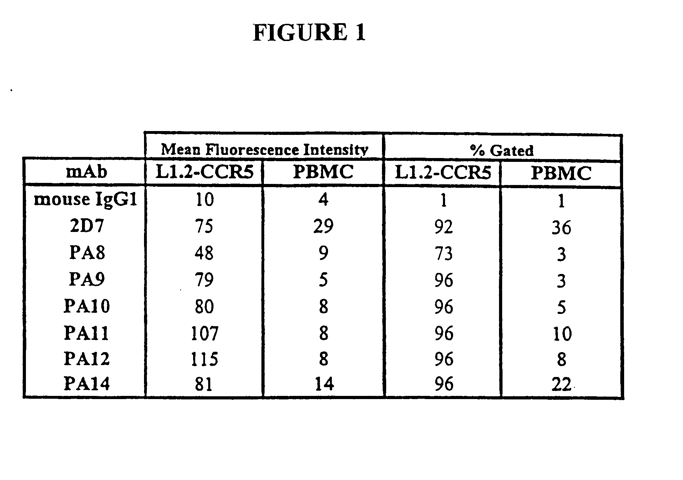

None of the purified anti-CCR5 mAbs stained the parental L1.2 cell line (data not shown). However, mAbs PA9-PA12 and PA14 stained >90%, and PA8 stained -70%, of L1.2-CCR5$^+$ cells as determined by flow cytometry, showing they recognized CCR5 (Fig. 1). The anti-CCR5 mAb 2D7, which was a positive control in our experiments, also stained >90% of L1.2-CCR5$^+$ cells. PA8-PA12 and PA14 are all IgG1, and react equally well with a goat anti-mouse IgG, whereas 2D7 is an IgG2a and may react differently with the reporter antibody. Only mean fluorescence intensities (m.f.i.) measured with mAbs PA8-PA12 and PA14 therefore are directly comparable. The rank order of mean fluorescence intensities (m.f.i.) was PA12- PA11> (2D7=) PA14~ PA10- PA9> PA8. The difference between PA12 m.f.i. and PA8 m.f.i. was three-fold. Differences in staining intensity between PA8 and the other mAbs remained constant over a wide range of concentrations (data not shown) and probably do not correspond to differences in mAb affinities for CCR5. This implies that PA8 interacts only with a subset of CCR5 molecules present on the surface of L1.2-CCR5$^+$ cells.
Compared with L1.2-CCR5+ cells, mitogen-stimulated PBMC exhibited different patterns of staining by the anti-CCR5 mAbs. 2D7 and PA14 stained >20%, PA11 and PA12 stained ~10%, PA8, PA9 and PA10 stained <5% of PBMC (Fig. 1). The mean fluorescence intensities of the stained PBMC were about tenfold lower than those obtained with L1.2-CCR5$^+$ cells for each mAb; their rank order was (2D7>) PA14> PA12- PA11~ PA10~ PA9-PA8. Again, this differed somewhat from the order of reactivities observed on CCR5 transfectants. The difference between PA9 m.f.i. and PA14 m.f.i. was seven-fold. Other groups have observed similar differences in the ability of anti-CCR5 mAbs to stain stable, CCR5$^+$ cell lines versus PBMC (28). This may be due to cell-specific differences in CCR5 conformation, post-translational modification or oligomerization. Alternatively, association with other cell surface molecules may differ between cells. Since an obvious choice for such a molecule would be the CD4 cell surface antigen, which is absent from L1.2-CCR5$^+$ cells and present on PBMCs, we also tested the ability PA8-PA12, PA14 and 2D7 to stain HeLa cells transiently expressing CCR5 alone or with CD4. No differences were observed in the ability of any of the mAbs to stain cell surface CCR5 in the presence of CD4 (data not shown). If there is an association between these two proteins, it does not involve epitopes recognized by the anti-CCR5 mAbs available to us. Alternatively, an association between CCR5 and CD4 might only occur on primary lymphocytes.

3) Epitope mapping of the mAbs using CCR5 alanine mutants

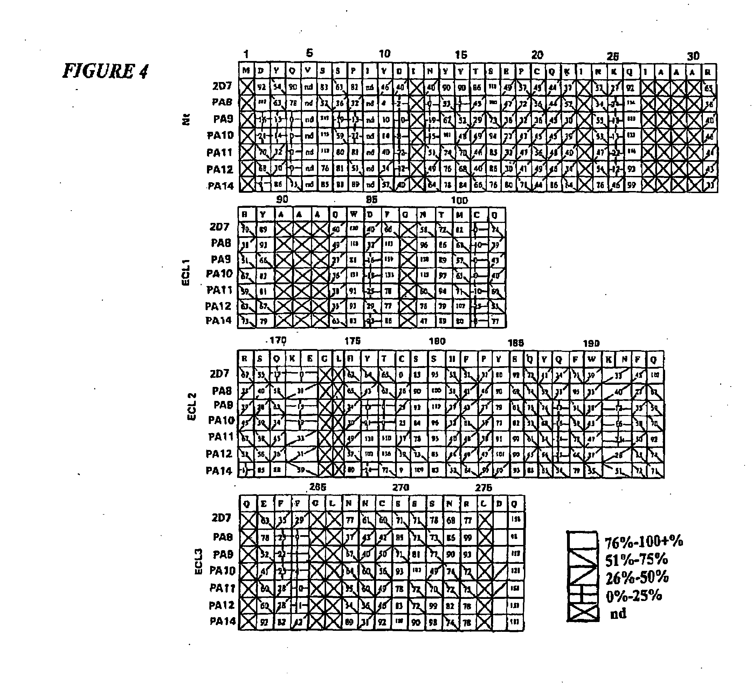

None of the antibodies were able to detect reduced and denatured CCR5 protein by Western blotting indicating that they recognize conformationally sensitive epitopes (data not shown). MAb epitope mapping studies were performed using a panel of seventy alanine point mutants of residues in the Nt and ECLs of CCR5. HeLa cells were lipofected with mutant or wild type CCR5 coding sequences appended with C-terminal HA tags, and infected with vTF7 (23) to boost co-receptor expression. The cells were then incubated with the anti-CCR5 mAbs and their binding was revealed by a PE-labeled goat anti-mouse IgG. A second, intracellular stain was performed with a FITC-labeled anti-HA mAb (BabCo). This internal control allowed us to directly normalize staining by the anti-CCR5 mAbs for mutant co-receptor expression levels on the cell surface. Hence, mAb binding to each mutant is expressed as a percentage of binding to wild-type CCR5 (Fig. 4).

Certain point mutations reduced the binding of all of the antibodies to CCR5 by >50%. In general, PA8-PA12 were the most affected, PA14 and 2D7 the least affected by this class of mutants, which included the cysteine pair C101A and C178A, the Nt mutants Y10A, D11A, K25A, the ECL1 mutant D95A, the ECL2 mutants K171A/E172A, Q188A, K191A/N192A, and the ECL3 mutants F263A and F264A (Fig. 4). One interpretation is that these residues are not part of the mAb epitopes *per se*, but that changing them to alanines causes conformational perturbations that have a common effect on binding of all mAbs. We assumed that if a mutation lowered binding of an individual mAb by >75%, and did not also lower binding of most of the other antibodies, the residue was probably a direct contributor to the epitope recognized by the mAb. Using these stringent guidelines, it was concluded that the seven anti-CCR5 mAbs recognize overlapping but distinct epitopes (Fig. 4). MAb PA8 binding to CCR5 depended on N13 and Y15 in the Nt. MAb PA9 and PA10 required D2, Y3, Q4, P8 and N13 in the Nt, and Y176 and T177 in ECL2. MAb PA9 also required S7 in the Nt. MAb PA11 and PA12 binding depended on Q4 in the Nt. PA14 required D2 in the Nt, and R168 and Y176 in ECL2. Finally, mAb 2D7 required Q170 and K171/E172 in ECL2 in order to bind to CCR5.

### 4) Chemokine signaling in the presence of anti-CCR5 mAbs

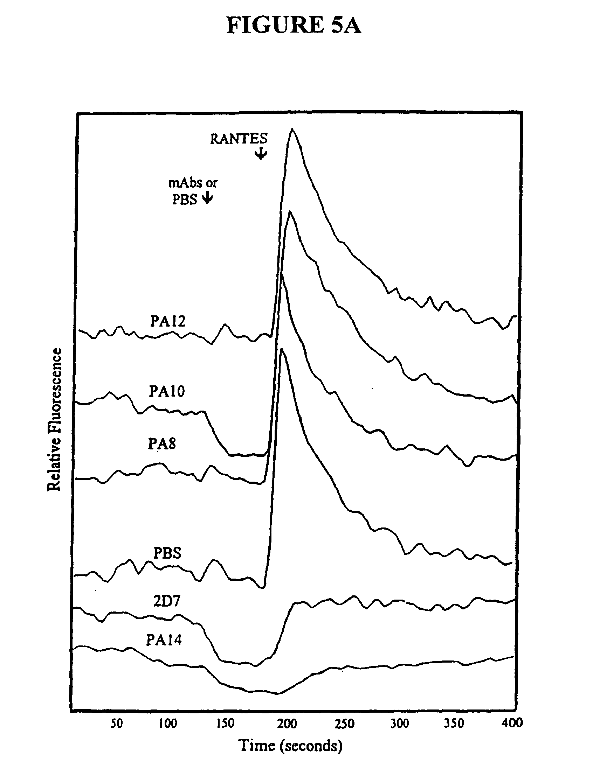

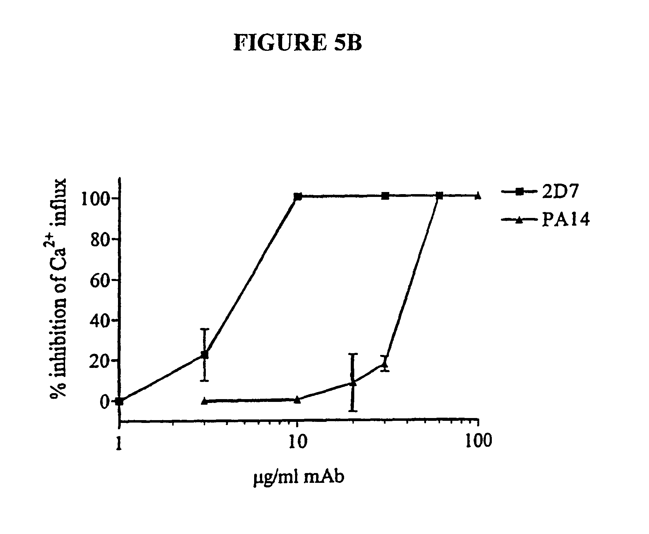

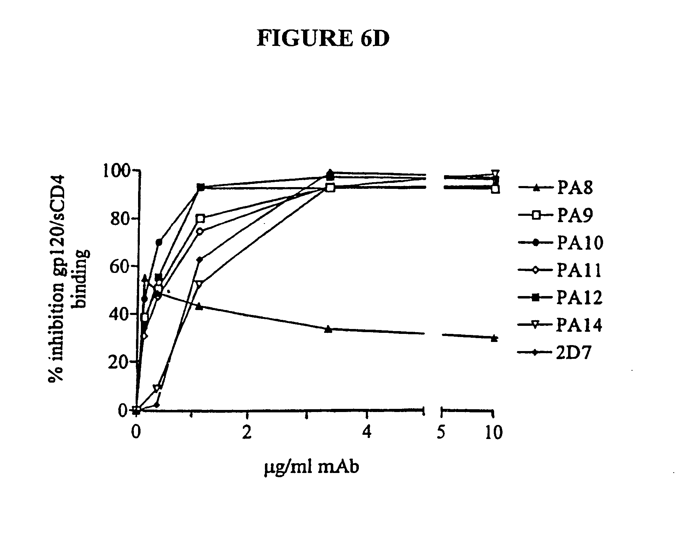

Chemokine receptor-binding agents can be antagonists or, more rarely, agonists of receptor-mediated intracellular signaling. Alternatively, they could have no effect on signaling. CCR5 is able to bind three CC-chemokines, RANTES, MIP-1• and MIP-1•, and transduce a signal that modulates cytosolic calcium levels. We therefore tested the agonist/antagonist activity of various concentrations of mAbs PA8-PA12, PA14 and 2D7. Changes in intracellular calcium concentrations, $(Ca^{2+})$ i, were measured in Indo-1-loaded L1.2-CCR5$^+$ cells. None of the mAbs stimulated a change in $(Ca^{2+})$ i, indicating that they are not agonists for CCR5. PA8-PA12 were also unable to inhibit $Ca^{2+}$ fluxes induced by RANTES (Fig. 5A and data not shown), even at concentrations as high as 100•g/ml, showing they are not antagonists either. These concentrations provide saturating binding of the mAbs to L1.2-CCR5$^+$ cells, as shown by flow cytometry and the gp120/CCR5 binding assay (Fig. 6D and data not shown). MAbs PA14 and 2D7, however, blocked calcium mobilization induced by RANTES, although with different potencies (Fig. 5A, B). The $IC_{50}$ for PA14 calcium influx inhibition was 50•g/ml, which was approximately 8-fold higher than the $IC_{50}$ for 2D7 (Fig. 5B). RANTES-, MIP-1•- and MIP-1•- induced calcium fluxes were each inhibited by similar concentrations of PA14 (data not shown). None of the mAbs affected SDF-1-induced calcium mobilization in L1.2-CCR5$^+$ cells, which endogenously express CXCR4 (data not shown). Finally, neither mAbs nor CC-chemokines affected cytosolic calcium levels in parental L1.2 cells (data not shown).

### 5) Inhibition of CCR5 co-receptor function by the mAbs

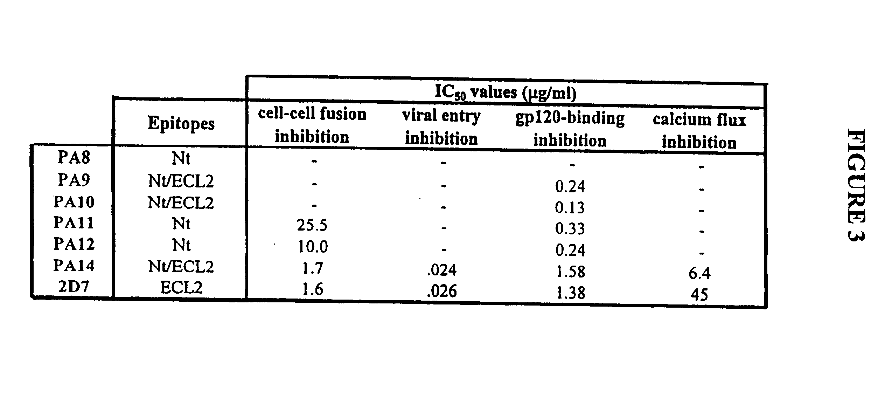

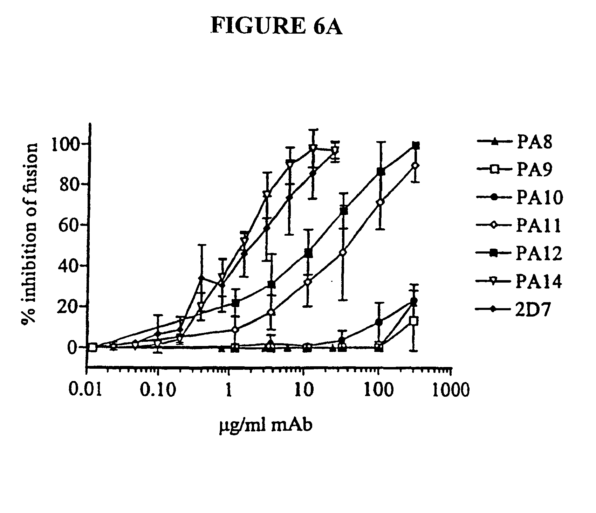

MAbs PA8-PA12 and PA14 were initially selected on the basis of their ability to inhibit HIV-1 envelope-mediated cell-cell fusion. This activity was confirmed and quantified for the purified mAbs. As expected, all six mAbs, as well as mAb 2D7, blocked fusion between CD4$^+$CCR5$^+$ PM1 cells and HeLa-Env$_{JR-FL}$$^+$ cells in the RET assay. The rank order of potency was 2D7-PA14> PA12> PA11> PA10~ PA9- PA8 (Fig. 6A). IC50 values for PA14 and 2D7 were 1.7•g/ml and 1.6•g/ml respectively, for PA11 and PA12 these were 25.5•g/ml and 10.0•g/ml respectively (Fig. 3). PA8, PA9 and PA10 inhibited fusion by only 10-15% at 300•g/ml. None of the mAbs affected fusion between PM1 cells and HeLa-Env$_{LAI}$$^+$ cells, which express the full length envelope protein from an X4 virus (data not shown).

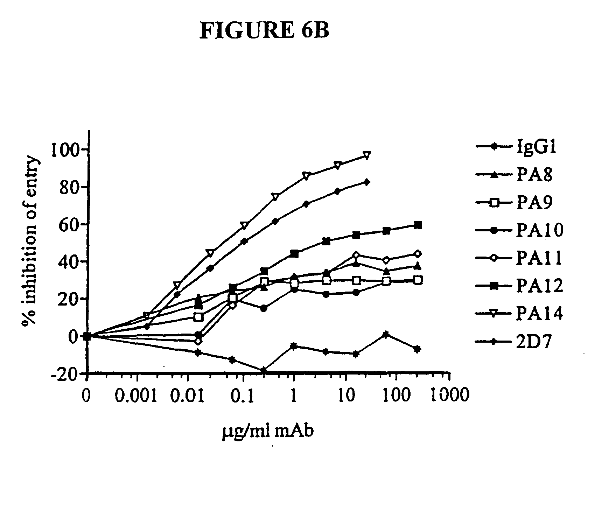

The ability of the different anti-CCR5 mAbs to inhibit entry of a prototypic R5 virus, JR-FL, and a R5X4 virus, Gun-1, in a single-round of replication, luciferase-based entry assay was also tested. The rank order of potency in the entry assay was similar to the one determined in the cell-cell fusion assay (Fig. 6B). A >50% inhibition of JR-FL or Gun-1 entry with PA8-PA11 was unable to be obtained. The $IC_{50}$ value for PA12 was 2.5 •g/ml. However, inhibition of entry by >60% with this mAb was unable to be obtained. The $IC_{50}$ values for PA14 and 2D7 inhibition of JR-FL entry were determined to be 0.024 and 0.026 •g/ml respectively (Fig. 3), and were 60-fold lower then those obtained in the fusion assay. Entry of dual-tropic Gun-1 was 2-3-fold more sensitive to inhibition by anti-CCR5 mAbs than JR-FL entry (data not shown).

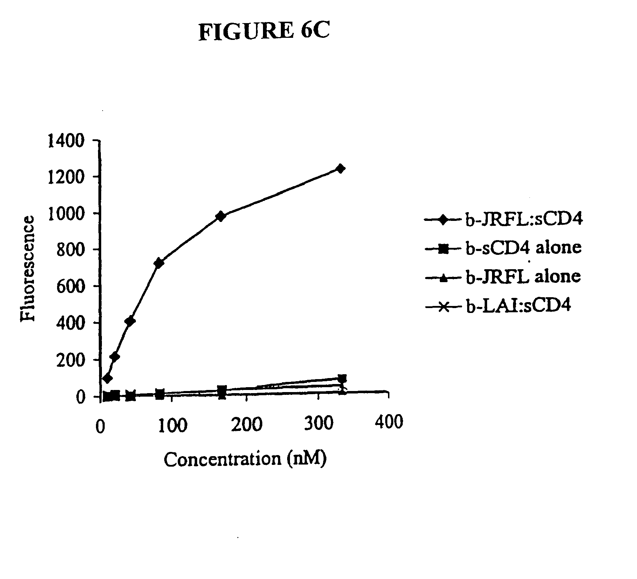

Anti-co-receptor mAbs might inhibit envelope-mediated fusion either by directly affecting the gp120/CCR5 interaction or by impeding post-binding steps involved in the formation of an active fusion complex. To determine the mechanism of inhibition of viral fusion and entry by PA8-PA12 and PA14, the ability of the different mAbs to block the gp120/CCR5 interaction was tested. For this an assay that detects binding to L1.2-CCR5$^+$ cells of biotinylated HIV-1$_{JR-FL}$ gp120 complexed with sCD4 was used. No binding of biotinylated gp120 was observed in the absence of sCD4 or CCR5, or when HIV-1$_{LAI}$ gp120 was used (Fig. 6C).

With the exception of PA8, all mAbs abrogated gp120/sCD4 binding to L1.2-CCR5$^+$ (Fig. 6D). Inhibition by PA8 saturated at -40%, which concurs with flow cytometry data (Fig. 1) in suggesting that this mAb binds only to a subset of CCR5 molecules on L1.2-CCR5$^+$ cells. MAbs PA9, PA10, PA11 and PA12 inhibited binding with IC$_{50}$ values of 0.24, 0.13, 0.33, 0.24μ g/ml respectively (Fig. 3). Surprisingly, mAbs PA14 and 2D7 were the two least efficient inhibitors of gp120/sCD4 binding, with IC$_{50}$ values of 1.58 and 1.38 •g/ml respectively (Fig. 3). Therefore, there was no correlation between the ability of a mAb to inhibit gp120/CD4/CCR5-mediated membrane fusion and entry and its ability to block gp120/sCD4 binding to the co-receptor.

6) <u>Synergistic inhibition of HIV-1 fusion by combinations of anti-CCR5 mAbs and other viral entry inhibitors</u>

Co-receptor-specific agents may act at multiple stages of the entry process and exhibit non-additive effects when used in combination. From a clinical perspective, it is important to determine the interactions of co-receptor-specific drug candidates with endogenous chemokines, which may afford some level of protection against disease progression. CCR5 mAbs were therefore tested in combination with each other or with RANTES, or with CD4-IgG2, which binds to HIV-1 gp120 to inhibit attachment to target cells. Dose-response curves were obtained for the agents used individually and in combination in viral fusion and entry assays. Data were analyzed using the median effect principle (9). The concentrations of single-agents or their mixtures required to produce a given effect were quantitatively compared in a term known as the Combination Index (CI). A CI value greater than 1 indicates antagonism, CI - 1 indicates an additive effect, and CI < 1 indicates a synergistic effect wherein the presence of one agent enhances the effect of another.

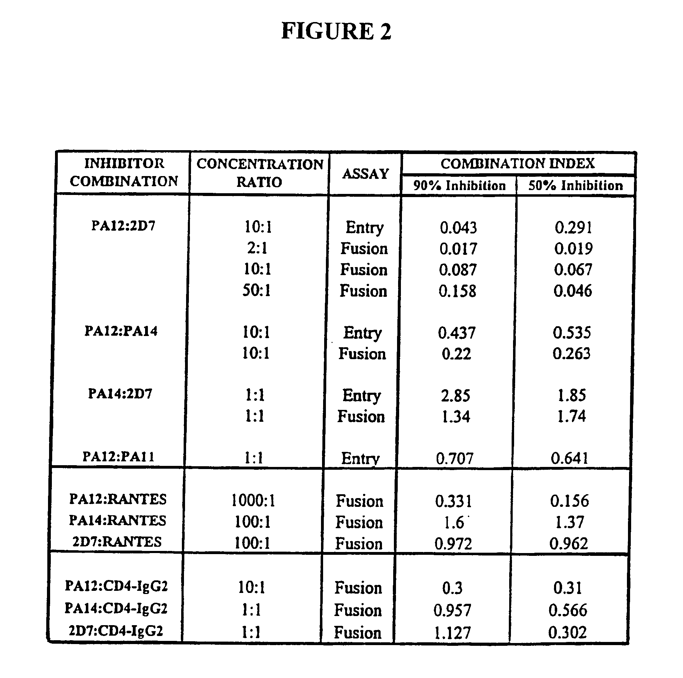

Combinations of PA12 and 2D7 were the most potently synergistic, with CI values ranging between 0.02 and 0.29, depending on the ratio of the antibodies (Fig. 7 and Fig. 2). The degree of synergy is known to vary with the stoichiometry of the agents. The viral entry and fusion assays were generally consistent in identifying mAb combinations that are highly synergistic, PA12 and 2D7; moderately synergistic, PA12 and PA14; additive, PA11 and PA12; and weakly antagonistic, PA14 and 2D7. The lack of synergy between PA14 and 2D7 is not surprising given that these mAbs cross-compete for binding to CCR5$^+$ cells as determined by flow cytometry (data not shown). The observation of an additive effect of PA11 and PA12 may be an indication that these mAbs bind to slightly different epitopes in CCR5, while sharing a dependency on residue Q4 in the Nt.

The ability of mAbs PA12, PA14 and 2D7 to synergize with RANTES in blocking cell-cell fusion was also tested. PA12 and RANTES combinations exhibited moderate synergy (Fig. 2). PA14 and 2D7 exhibited no synergy with RANTES, which is consistent with these mAbs being inhibitory of RANTES binding and signaling (Fig. 5A, B). Finally, we tested synergy between mAbs PA12, PA14, 2D7 and CD4-IgG2, which interacts with gp120. We observed moderate synergy between PA12 and CD4-IgG2 but no synergy between PA14 or 2D7 and CD4-IgG2 (Fig. 2).

**Experimental Discussion**

Six murine anti-CCR5 IgG1 mAbs were isolated and characterized. Whereas PA8, PA9, PA11, PA12 and PA14 are distinct molecular species, PA9 and PA10 are indistinguishable by the analyses and therefore are probably the same mAb. All of the mAbs that were isolated recognize complex conformational epitopes, as is often the case with mAbs raised against native, cell surface proteins. Epitope mapping was performed for all mAbs using a panel of CCR5 alanine point mutants. Residues that affected binding of all mAbs similarly were assumed to cause conformational perturbations in the co-receptor and not to constitute part of the mAb epitopes. Only two such residues, Y10 and D11, have been shown to affect HIV-1 entry (20, 52). The PA8, PA11 and PA12 epitopes are located exclusively in the Nt domain. Consistent with this result, PA8 was able to bind a biotinylated Nt peptide, containing residues D2 through R31, in an ELISA (data not shown). However, PA11 and PA12, whose binding strongly depended only on Q4, did not bind the Nt peptide in solution (data not shown). One possibility is that the Nt peptide does not assume the proper conformation for recognition by PA11 and PA12, whereas PA8 binding may be less conformation-dependent. Alternatively, PA11 and PA12 might interact with residues that we have not mutated, or form weak bonds with amino acids located in other domains of CCR5, or bind peptide backbone atoms whose presentation may be unchanged by mutagenesis. Antibodies PA9, PA10 and PA14 recognized epitopes that included residues in both the Nt and ECL2 domains of CCR5, whereas the 2D7 epitope was located exclusively in ECL2.

The PA14 epitope comprises both D2 in the Nt and R168 in ECL2 indicating that these two residues are proximal to one another within the context of a mAb footprint. They may even directly interact with one another through their opposite charges.

MAbs PA8-PA12 and PA14 stained CCR5$^+$ cells with different intensities and in a cell type-dependent manner. All mAbs except PA8 stained >90% L1.2-CCR5$^+$ cells, the highest mean fluorescence intensity being observed with PA11 and PA12. However, PA14 and 2D7 stained the highest percentage of PBMC and also yielded the highest mean fluorescence intensities on these cells. Hill et al. (28) have recently characterized a panel of anti-CCR5 mAbs that similarly stained transfected cells, but only two of eight stained PBMC, and none stained primary monocytes. A low affinity for CCR5 probably accounted for the non-reactivity of two of the mAbs with primary cells, but this was unlikely to be the explanation for the failure of the other four to react. In our mAb panel, we observe the most intense staining of PBMC by mAbs 2D7 and PA14 that have epitopes located entirely or partially in the first ten residues of ECL2. Hill et al. report, however, that mAbs specific for the Nt and ECL1 stain PBMCs, while mAbs to ECL2 and ECL3 do not stain PBMC, so a consistent pattern of reactivity has not been identified. One explanation for cell type-specific staining by mAbs would be that activated PBMCs (and monocytes) secrete CC-chemokines that bind to cell surface CCR5, masking some mAb epitopes. However, one would expect this to be especially true for PA14 and 2D7, which are antagonists of chemokine-induced calcium mobilization and presumably compete with CC-chemokines for binding to CCR5. Yet these mAbs stain PBMC the most intensely. Alternatively, differential CCR5 epitope exposure may reflect cell type-specific receptor oligomerization, association with other cell-surface molecules, or different post-translational modifications such as glycosylation. We have shown that differences in mAb binding probably do not reflect cell type-specific differences in CD4/CCR5 interactions.

MAbs PA8-PA12 did not inhibit CC-chemokine induced calcium mobilization in CCR5$^+$ cells, nor did they mediate signaling through CCR5. MAbs 2D7 and PA14 were inhibitors of CC-chemokine induced calcium mobilization, but 2D7 was almost an order of magnitude more potent than PA14. This may be because the PA14 epitope overlaps less with the CC-chemokine binding domain on CCR5 than the 2D7 epitope. All of the mAbs also blocked HIV-1 entry and envelope-mediated membrane fusion, but inhibition of cell-cell fusion required in some cases almost two orders of magnitude more antibody than what was needed to block viral entry. Presumably, more gp120/CD4/CCR5 interactions as well as interactions between adhesion molecules are established and act cooperatively during cell-cell fusion, compared to virus-cell fusion, making it more difficult to inhibit. This is commonly observed with antibodies to LFA-1 or to the HIV-1 envelope glycoprotein (45, 51). PA8, PA9 and PA10 were unable to block cell-cell fusion by >15% and viral entry by >40%, even at the highest antibody concentrations. However, >90% inhibition of fusion could be attained with PA11, PA12 and PA14, and >90% inhibition of entry could be attained with PA14. The most potent of the six mAbs in blocking fusion and entry was PA14, which was as effective as 2D7. Surprisingly, PA14 and 2D7 were among the least potent inhibitors of gp120/sCD4 binding to L1.2-CCR5$^+$ cells, whereas PA9-PA12 blocked with similar potencies, and PA8 was unable to block >40% of gp120/sCD4 binding. These observations raise questions about the nature of the CCR5 molecules presented on different cells and about the mechanisms of inhibition of viral fusion and entry. It may be that CCR5 on L1.2 cells, used in the mAb and gp120-binding assays, is not in an identical conformation to CCR5 on PBMC, used in the mAb-binding assay, or to CCR5 on PM1 and U87MG cells used in the fusion and entry assays.

The low staining of PBMC and the partial inhibition of fusion and entry by some of our mAbs indicate that they are only able to bind to a subset of CCR5 molecules expressed on primary lymphocytes, PM1 and U87MG-CD4$^+$CCR5$^+$ cell lines. Yet, other than PA8, all mAbs are able to stain >90% L1.2-CCR5$^+$ cells and to completely block binding of the gp120/sCD4 complex to these cells. At least one difference between L1.2-CCR5$^+$ and the other cells that we have used is the density of co-receptor protein on the cell surface. Indeed, we estimate that the L1.2-CCR5$^+$ cells express 10- to 100-fold more cell surface co-receptor than PM1 and U87MG-CD4$^+$CCR5$^+$ cells. But when HeLa cells are engineered to transiently express as much co-receptor as the L1.2-CCR5$^+$ cell line, we are still unable to detect gp120/sCD4 binding to them (data not shown). Over-expression of CCR5 on L1.2, along with other cell-specific factors therefore, might favor a co-receptor conformation that prominently exposes the Nt, making it more accessible to both mAbs and gp120. Such a conformation might be induced by receptor oligomerization, by diminished or altered associations with cell surface proteins or by receptor interactions with G proteins (25, 62). Do multiple conformations of CCR5 co-exist on the cell surface, and are they all permissive for viral entry? The patterns of mAb reactivity would suggest so, since HIV-1 entry and fusion can occur, albeit at reduced levels, in the presence of mAb concentrations that saturate epitopes required for gp120 binding to L1.2-CCR5+ cells. We favor the hypothesis that the co-receptor molecules present on L1.2-CCR5$^+$ cells possess one HIV-1 entry-competent conformation whereas CCR5 molecules on PBMC, PM1 and CCR5$^+$ U87MG exist in multiple, entry-competent states that display different mAb reactivities. Whereas PA14 and 2D7 may recognize all conformations, other mAbs may not. Why L1.2 cells are conducive to a particular fusion-competent conformation remains to be determined.

It has recently been demonstrated that the gp120-binding domain lies in the first twenty residues of the CCR5 Nt domain. MAbs to the gp120-binding domain on CCR5 potently block this interaction but are not nearly as efficient at inhibiting HIV-1 fusion and entry into target cells as PA14 and 2D7, whose epitopes lie outside this region. PA14 recognizes the tip of the Nt and residues in ECL2, whereas the 2D7 epitope is located exclusively in ECL2. At the mechanism of action of these mAbs can only be speculated. It may be that their binding to the first few residues of ECL2 induces conformational changes in the co-receptor that prevent membrane fusion. Alternatively, obstruction of ECL2 epitopes might impede co-receptor oligomerization and the formation of a fusion-competent protein complex. Yet another possibility is that residues in ECL2 face the inside of the fusion pore and binding of the mAbs impedes gp41 from inserting the fusion peptide into the plasma membrane. In contrast, mAbs PA8-PA12 probably inhibit fusion and entry only by directly competing for binding with gp120/CD4 complexes. We do not know if parameters other than epitope exposure and affinity for CCR5 determine the efficacy of viral entry inhibition by these mAbs. It is unclear why inhibiting steps subsequent to the gp120/co-receptor interaction would be more efficient than directly blocking that interaction. One way to explain this would be to assume that the off rate of gp120 binding to CCR5 is much lower than the on rate of mAb binding to CCR5. Thus, every time a mAb detaches itself from a co-receptor molecule, a virion-associated gp120 molecule replaces it in a quasi-irreversible fashion since this interaction leads to membrane fusion.

Synergy between combinations of anti-CCR5 mAbs is probably a result of their interactions with distinct epitopes that are involved in inter-dependent, consecutive steps of HIV-1 entry. The degree of synergy observed between PA12 and 2D7 (CI<0.1 under many circumstances) is extraordinary since CI values <0.2 are rarely observed for combinations of anti-HIV-1 antibodies (33, 35, 61), reverse transcriptase inhibitors (29), or protease inhibitors (44). Because of its potency, the PA12:2D7 combination was examined in multiple assay formats and concentration ratios, for which consistently high levels of synergy were observed. Moderate synergy was observed for PA12 combined with PA14. We also observed moderate synergy between PA12 and CD4-IgG2. The CD4/gp120 complex is metastable and if it is unable to interact with a co-receptor, decays into a non-fusogenic state (45-48). Since PA12 directly blocks the gp120-binding site on CCR5, its presence may shift the equilibrium towards inactivation of the gp120/CD4 complex. This would explain why we observe synergy between CD4-IgG2 and mAb PA12 with respect to inhibition of fusion and entry. The lack of synergy between mAb PA14 and CD4-IgG2 suggests that they act on two nonconsecutive and independent steps of viral entry. A combination of further studies will be needed to determine the precise mechanisms of synergy of the different compounds with respect to inhibition of viral fusion and entry.

The above results are consistent with a model wherein HIV-1 entry occurs in three distinct steps involving receptor binding, co-receptor binding, and co-receptor mediated membrane fusion. Separate co-receptor binding and fusion events are suggested by the lack of correlation between the monoclonal antibodies' abilities to block gp120 binding and HIV-1 fusion/entry. The chronology of events during fusion is further suggested by the patterns of synergies observed. Agents, such as PA12, that potently inhibit the middle step of the process, namely gp 120 binding, act synergistically with inhibitors of prior and subsequent steps.

REFERENCES

1. Allaway, G.P., K.L. Davis-Bruno, B.A. Beaudry, E.B. Garcia, E.L. Wong, A.M. Ryder, K.W. Hasel, M.C. Gauduin, R.A. Koup, J.S. McDougal and P.J. Maddon. 1995. Expression and characterization of CD4-IgG2, a novel heterotetramer that neutralizes primary HIV type 1 isolates. AIDS Res Hum Retroviruses 11: 533-539.

2. Allaway, G.P., A.M. Ryder, G.A. Beaudry and P.J. Maddon. 1993. Synergistic inhibition of HIV-1 envelope-mediated cell fusion by CD4-based molecules in combination with antibodies to gp120 or gp41. AIDS Res Hum Retroviruses 9: 581-587.

3. Amara, A., S.L. Gall, O. Schwartz, J. Salamero, M. Montes, P. Loetscher, M. Baggiolini, J.L. Virelizier and F. Arenzana-Seisdedos. 1997. HIV coreceptor downregulation as antiviral principle: SDF-1a-dependent internalization of the chemokine receptor CXCR4 contributes to inhibition of HIV replication. J. Exp. Med. 186: 139-146.

4. Berger, E.A. 1997. HIV entry and tropism: the chemokine receptor connection. AIDS 11 (suppl A): S3-S16.

5. Bieniasz, P.D. and B.R. Cullen. 1998. Chemokine receptors and human immunodeficiency virus infection. Frontiers in Bioscience 3: d44-58.

6. Bieniasz, P.D., R.A. Fridell, I. Aramori, S.S.G. Ferguson, M.C. Caron and B.R. Cullen. 1997. HIV-1-induced cell fusion is mediated by multiple regions within both the viral envelope and the CCR5 co-receptor. EMBO 16: 2599-2609.

7. Brelot, A., N. Heveker, O. Pleskoff, N. Sol and M. Alizon. 1997. Role of the first and third extracellular domains of CXCR4 in human immunodeficiency virus coreceptor activity. J. Virol. 71: 4744-4751.

8. Chan, D.C. and P.S. Kim. 1998. HIV entry and its inhibition. Cell 93: 681-684.

9. Chou, T.C. and D.C. Rideout. Synergism and antagonism in chemotherapy. New York: Academic Press, 1991

10. Cocchi, F., A.L. DeVico, A. Garzino-Derno, S.K. Arya, R.C. Gallo and P. Lusso. 1995. Identification of RANTES, MIP-1$\alpha$ and MIP-1$\beta$ as the major HIV-suppressive factors produced by CD8 T-cells. Science 270: 1811-1815.

11. Connor, R.I., K.E. Sheridan, D. Ceradini, S. Choe and N.R. Landau. 1997. Change in co-receptor use correlates with disease progression in HIV-1 infected individuals. J. Exp. Med. 185: 621-628.

12. Crump, M.P., J.H. Gong, P. Loetscher, K. Rajarathnam, A. Amara, F. Arenzana-Seisdedos, J.L. Virelizier, M. Baggiolini, B.D. Sykes and I. Clark-Lewis. 1997. Solution structure and basis for functional activity of stromal-cell derived factor-1; disassociation of CXCR4 activation from binding and inhibition of HIV-1. EMBO 16: 6996-7007.

13. Dalgleish, A.G., P.C.L. Beverly, P.R. Clapham, D.H. Crawford, M.F. Greaves and R.A. Weiss. 1984. The CD4 (T4) antigen is an essential component of the receptor for the AIDS retrovirus. Nature 312: 763-766.

14. Deng, H.K., R. Liu, W. Ellmeier, S. Choe, D. Unutmaz, M. Burkhart, P. DiMarizio, S. Marmon, R.E. Sutton, C.M. Hill, S.C. Peiper, T.J. Schall, D.R. Littman and N.R. Landau. 1996. Identification of a major co-receptor for primary isolates of HIV-1. Nature 381: 661-666.

15. Dimitrov, D.S. 1997. How do viruses enter cells? The HIV Co-receptors teach us a lesson of complexity. Cell 91: 721-730.

16. Donzella, G.A., D. Schols, S.W. Lin, K.A. Nagashima, P.J. Maddon, G.P. Allaway, T.P. Sakmar, E.D. Clercq and J.P. Moore. 1998. JM3100, a small molecule that interacts with the CXCR4 co-receptor to prevent HIV-1 entry. Nat. Med. 4: 72-77.

17. Doranz, B.J., K. Grovit-Ferbas, M.P. Sharron, S.H. Mao, M.B. Goetz, E.S. Daar, R.W. Doms and W.A. O'Brien. 1997. A small molecule inhibitor directed against the chemokine receptor CXCR4 prevents its use as an HIV-1 co-receptor. J. Ex. Med. 186: 1395-1400.

18. Doranz, B.J., Z.-H. Lu, J. Rucker, T.-Y. Zhang, M. Sharron, Y.-H. Cen, Z.-X. Wang, H.-H. Guo, J.-G. Du, M.A. Accavitti, R.W. Doms and S.C. Peiper. 1997. Two distinct CCR5 domains can mediate co-receptor usage by human immunodeficiency virus type 1. J. Virol. 71: 6305-6314.

19. Dragic, T., V. Litwin, G.P. Allaway, S.R. Martin, Y. Huanh, K.A. Nagashima, C. Cayanan, P.J. Maddon, R.A. Koup, J.P. Moore and W.A. Paxton. 1996. HIV-1 entry into CD4+ cells is mediated by the chemokine receptor CC-CKR-5. Nature 381: 667-673.

20. Dragic, T., A. Trkola, X.W. Lin, K.A. Nagashima, F. Kajumo, L. Zhao, W.C. Olson, L. Wu, C.R. Mackay, G.P. Allaway, T.P. Sakmar, J.P. Moore and P.J. Maddon. 1998. Amino terminal substitutions in the CCR5 co-receptor impair gp120 binding and human immunodeficiency virus type 1 entry. J. Virol. 72: 279-285.

21. Dragic, T., A. Trkola and J.P. Moore. 1997. HIV co-receptors: Gateways to the cell. Advances in Research and Therapy 7: 2-13.

22. Farzan, M., H. Choe, L. Vaca, K. Martin, Y. Sun, E. Desjardins, N. Ruffing, L. Wu, R. Wyatt, N. Gerard, C. Gerard and J. Sodroski. 1998. A tyrosine-rich region in the N-terminus of CCR5 is important for human immunodeficiency virus type 1 entry and mediates an association between gp120 and CCR5. J. Virol. 72: 1160-1164.

23. Fuerst, T.R., E.G. Niles, F.W. Studier and B. Moss. 1986. Eukaryotic transient-expression system based on recombinant vaccinia virus that synthesizes bacteriophage T7 RNA polymerase. Proc. Natl. Acad. Sci. USA. 83: 8122-8126.

24. Genoud, S., F. Kajumo, Y. Guo, D.A.D. Thompson and T. Dragic. CCR5-mediated human immunodeficiency virus entry depends on an amino-terminal domain gp120-binding site and on the conformational integrity of all four extracellular domains. J. Virol. submitted.

25. Gether, U. and B.K. Kobilka. 1998. G protein-coupled receptors. J. Biol. Chem 273: 17979-17982.

26. Gordon, C., M. Muesing, A.E.I. Proudfoot, C.A. Power, J.P. Moore and A. Trkola. 1998. Enhancement of human immunodeficiency virus type 1 infection by the CC-chemokine RANTES is independent of the mechanism of virus-cell fusion. J. Virol. in press.

27. Heveker, N., M. Montes, L. Germeroth, A. Amara, A. Trautmann, M. Alizon and J. Schneider-Mergener. 1998. Dissociation of the signaling and antiviral properties of SDF-1-derived small peptides. Current Biology 8: 369-376.

28. Hill, C.M., D. Kwon, M. Jones, C.B. Davis, S. Marmon, B.L. Daugherty, J.A. DeMartino, M.S. Springer, D. Unutmaz and D.R. Littman. 1998. The amino terminus of human CCR5 is required for its function as a receptor for diverse human and simian immunodeficiency virus envelope glycoproteins. Virology 248: 357-371.

29. Johnson, V.A., D.P. Merrill, J.A. Videler, T.C. Chou, R.E. Byington, J.J. Eron, R.T. D'Aquila and M.S. Hirsch. 1991. Two-drug combinations of zidovudine, didanosine, and recombinant interferon-alpha A inhibit replication of zidovudine-resistant human immunodeficiency virus type 1 synergistically in vitro. J Infect Dis 164: 646-655.

30. Klatzmann, D., E. Champagne, S. Chamaret, J.M. Gruest, D. Guetard, T. Hercend, J.C. Gluckman and L. Montagnier. 1984. T-lymphocyte T4 molecule behaves as the receptor for human retrovirus LAV. Nature 312: 382-385.

31. Kuhmann, K.E., E.J. Platt, S.L. Kozak and D. Kabat. 1997. Polymorphism in the CCR5 genes of African green monkeys and mice implicate specific amino acids in infections by simian and human immunodeficiency viruses. J. Virol. 71: 8642-8656.

32. Kwong, P.D., R. Wyatt, J. Robinson, R.W. Sweet, J. Sodroski and W.A. Hendrickson. 1998. Structure of an HIV gp120 envelope glycoprotein in complex with the CD4 receptor and a neutralizing human antibody. Nature 393: 648-659.

33. Laal, S., S. Burda, M.K. Gorny, S. Karwowska, A. Buchbinder and S. Zolla-Pazner. 1994. Synergistic neutralization of human immunodeficiency virus type 1 by combinations of human monoclonal antibodies. J. Virol. 68: 4001-4008.

34. Labrosse, B., A. Brelot, N. Heveker, N. Sol, D. Schols, E.D. Clercq and M. Alizon. 1998. Determinants for sensitivity of human immunodeficiency virus co-receptor CXCR4 to the bicyclam AMD3100. J. Virol. 72: 6381-6388.

35. Li, A., H. Katinger, M.R. Posner, L. Cavacini, S. Zolla-Pazner, M.K. Gorny, J. Sodroski, T.C. Chou, T.W. Baba and R.M. Ruprecht. 1998. Synergistic neutralization of simian-human immunodeficiency virus SHIV-vpu+ by triple and quadruple combinations of human monoclonal antibodies and high-titer anti-human immunodeficiency virus type 1 immunoglobulins. J. Virol. 72: 3235-3240.

36. Littman, D.R. 1998. Chemokine receptors: keys to AIDS pathogenesis. Cell 93: 677-680.

37. Litwin, V. unpublished results.

38. Litwin, V., K. Nagashima, A.M. Ryder, C.H. Chang, J.M. Carver, W.C. Olson, M. Alizon, K.W. Hasel, P.J. Maddon and G.P. Allaway. 1996. Human immunodeficiency virus type 1 membrane fusion mediated by a laboratory-adapted strain and a primary isolate analyzed by resonance energy transfer. J. Virol. 70: 6437-6441.

39. Loetscher, P., J.H. Gong, B. Dewald, M. Baggioloni and I. Clark-Lewis. 1998. N-terminal peptides of stromal cell derived factor-1 with CXC chemokine receptor 4 agonist and antagonist activities. J. Biol. Chem. 273: 22279-22283.

40. Mack, M., B. Luckow, P.J. Nelson, J. Cihak, G. Simmons, P.R. Clapham, N. Signoret, M. Marsh, M. Stangassinger, F. Borlat, T.N.C. Wells, D. Schlondorff and A.E.I. Proudfoot. 1998. Aminooxypentane-RANTES induces CCR5 internalization but inhibits recycling: a novel inhibitory mechanisms of HIV infectivity. J. Ex. Med. 187: 1215-1224.

41. Maddon, P.J., A.G. Dalgleish, J.S. McDougal, P.R. Clapham, R.A. Weiss and R. Axel. 1986. The T4 gene encodes the AIDS virus receptor and is expressed in the immune system and the brain. Cell 47: 333-348.

42. McDougal, J.S., M.S. Kennedy, J.M. Sligh, S.P. Cort, A. Mawle and J.K.A. Nicholson. 1986. Binding of HTLVIII / LAV to T4+ T cells by a complex of the 110K viral protein and the T4 molecule. Science 231: 382-385.

43. McKnight, A., D. Wilkinson, G. Simmons, S. Talbot, L. Picard, M. Ahuja, M. Marsh, J.A. Hoxie and P.R. Clapham. 1997. Inhibition of human immunodeficiency virus fusion by a monoclonal antibody to a co-receptor (CXCR4) is both cell type and virus strain dependent. J. Virol. 71: 1692-1696.

44. Merrill, D.P., D.J. Manion, T.C. Chou and M.S. Hirsch. 1997. Antagonism between human immunodeficiency virus type 1 protease inhibitors indinavir and saquinavir in vitro. J Infect Dis 176: 265-268.

45. Moore, J.P., Y. Cao, L. Qing, Q.J. Sattentau, J. Pyati, R. Koduri, J. Robinson, C.F. Barbas, D.R. Burton and D.D. Ho. 1995. Primary isolates of human immunodeficiency virus type 1 are relatively resistant to neutralization by monoclonal antibodies to gp120 and their neutralization is not predicted by studies with monomeric gp120. J. Virol. 69: 101-109.

46. Moore, J.P., B.A. Jameson, R.A. Weiss and Q.J. Sattentau.The HIV-cell fusion reaction. Boca Raton: CRC Press Inc., 1993 (J. Bentz, ed. Viral Fusion Mechanisms)

47. Moore, J.P., J.A. McKeating, Y. Huang, A. Ashkenazi and D.D. Ho. 1992. Virions of primary human immunodeficiency virus type 1 isolates resistant to soluble CD4 (sCD4) neutralization differ in sCD4 binding and glycoprotein gp120 retention from sCD4-sensitive isolates. J. Virol. 66: 235-243.

48. Moore, J.P. and R.W. Sweet. 1993. The HIV gp120-CD4 interaction: a target for pharmacological and immunological intervention. Prospect in Drug Discovery and Design 1: 235-250.

49. Murakami, T., T. Nakajima, Y. Koyanagi, K. Tachibana, N. Fujii, H. Tamamura, N. Yoshida, M. Waki, A. Matsumoto, O. Yoshie, T. Kishimoto, N. Yamamoto and T. Nagasawa. 1997. A small molecule CXCR4 inhibitor that blocks T cell line-tropic HIV-1 infection. J. Ex. Med. 186: 1389-1393.

50. Olson, W.C. unpublished results.

51. Pantaleo, G., G. Poli, L. Butini, C. Fox, A.I. Dayton and A.S. Fauci. 1991. Dissociation between syncytia formation and HIV spreading. Suppression of syncytia does not necessarily reflect inhibition of HIV infection. Eur. J. Immunol. 21: 1771-1774.

52. Rabut, G.E.E., J.A. Konner, F. Kajumo, J.P. Moore and T. Dragic. 1998. Alanine substitutions of polar and non-polar residues in the amino-terminal domain of CCR5 differently impair entry of macrophage- and dual-tropic isolates of the human immunodeficiency virus type 1. J. Virol. 72: 3464-3468.

53. Rizzuto, C., R. Wyatt, N. Hernandez-Ramos, Y. Sun, P. Kwong, W. Hendrickson and J. Sodroski. 1998. Identification of a conserved human immunodeficiency virus gp120 glycoprotein structure important for chemokine receptor binding. Science 280: 1949-1953.

54. Rucker, J., M. Samson, B.J. Doranz, F. Libert, J.F. Berson, Y. Yi, R.J. Smyth, R.G. Collman, C.C. Broder, G. Vassart, R.W. Doms and M. Parmentier. 1996. Regions in the β-chemokine receptors CCR-5 and CCR-2b that determine HIV-1 cofactor specificity. Cell 87: 437-446.

55. Schols, D., S. Struyf, J.V. Damme, J.A. Este, G. Henson and E.D. Clercq. 1997. Inhibition of T-tropic HIV strains by selective antagonization of the chemokine receptor CXCR4. J. Ex. Med. 186: 1383-1388.

56. Simmons, G., P.R. Clapham, L. Picard, R.E. Offord, M.M. Rosenkilde, T.W. Schwartz, R. Buser, T.N.C. Wells and A.E.I. Proudfoot. 1997. Potent inhibition of HIV-1 infectivity in macrophages and lymphocytes by a novel CCR5 antagonist. Science 276: 276-279.

57. Simmons, G., D. Wilkinson, J.D. Reeves, M.T. Dittmar, S. Beddows, J. Weber, G. Carnegie, U. Desselberger, P.W. Gray, R.A. Weiss and P.R. Clapham. 1996. Primary, syncytium-inducing human immunodeficiency virus type-1 isolates are dual-tropic and most can use either LESTR or CCR5 as co-receptor for virus entry. J. Virol. 70: 8355-8360.

**EP 1 144 006 B1**

58. Strizki, J.M., J. Davis-Turner, R.G. Collman, J. Hoxie and F. Gonzalez-Scarano. 1997. A monoclonal antibody (12G5) directed against CXCR4 inhibits infection with the dual-tropic human immunodeficiency virus type 1 isolate HIV-1 89.6 but not the T-tropic isolate HIV-1 HxB. J. Virol. 71: 5678-5683.

59. Trkola, A., T. Dragic, J. Arthos, J. Binley, W.C. Olson, G.P. Allaway, C. Cheng-Mayer, J. Robinson, P.J. Maddon and J.P. Moore. 1996. CD4-dependent, antibody sensitive interactions between HIV-1 and its co-receptor CCR-5. Nature 384: 184-187.

60. Trkola, A., W.A. Paxton, S.P. Monard, J.A. Hoxie, M.A. Siani, D.A. Thompson, L. Wu, C.R. Mackay, R. Horuk and J.P. Moore. 1997. Genetic subtype-independent inhibition of human immunodeficiency virus type-1 replication by CC- and CXC chemokines. J. Virol. 72: 396-404.

61. Vijh-Warrier, S., A. Pinter, W.J. Honnen and S.A. Tilley. 1996. Synergistic neutralization of human immunodeficiency virus type 1 by a chimpanzee monoclonal antibody against the V2 domain of gp120 in combination with monoclonal antibodies against the V3 loop and the CD4-binding site. J. Virol. 70: 4466-4473.

62. Ward, S.G., K. bacon and J. Westwick. 1998. Chemokines and lymphocytes: more than an attraction. Immunity 9: 1-11.

63. Wu, L., N.P. Gerard, R. Wyatt, H. Choe, C. Parolin, N. Ruffing, A. Borsetti, A.A. Cardoso, E. Desjardin, W. Newman, C. Gerard and J. Sodroski. 1996. CD4-induced interaction of primary HIV-1 gp120 glycoproteins with the chemokine receptor CCR-5. Nature 384: 179-183.

64. Wu, L., G. LaRosa, N. Kassam, C.J. Gordon, H. Heath, N. Ruffing, H. Chen, J. Humblias, M. Samson, M. Parmentier, J.P. Moore and C.R. Mackay. 1997. Interaction of chemokine receptor CCR5 with its ligands: multiple domains for HIV-1 gp120 binding and a single domain for chemokine binding. J. Exp. Med. 186: 1373-1381.

65. Wyatt, R., P.D. Kwong, E. Desjardins, R. Sweet, J. Robinson, W. Hendrickson and J. Sodroski. 1998. The antigenic structure of the human immunodeficiency virus gp120 envelope glycoprotein. Nature 393: 705-711.

66. Wyatt, R. and J. Sodroski. 1998. The HIV-1 envelope glycoproteins: fusogens, antigens and immunogens. Science 280: 1884-1888.

67. Ylisastigui, L., J.J. Vizzavona, E. Drakopoulou, P. Paindavoine, C.F. Calvo, M. Parmentier, J.C. Gluckman, C. Vita and A. Benjouad. 1998. Synthetic full length and truncated RANTES inhibit HIV-1 infection of primary macrophages. AIDS 12: 977-984.

68. Zhang, J.L., H. Choe, B.J. Dezube, M. Farzan, P.L. Sharma, X.C. Zhou, L.B. Chen, M. Ono, S. Gillies, Y. Wu, J.G. Sodroski and C.S. Crumpacker. 1998. The bis-azo compound FP-21399 inhibits HIV-1 replication by preventing viral entry. Virology 244: 530-541.

69. Cairns, J.S., D'Souza. M.P., 1998. Chemokines and HIV-1 second receptors: the therapeutic connection. Nature Medicine. 1998. Vol 4, No. 5: 563.

70. Kilby, J.Michael, et al. 1998. Potent suppression of HIV-1 replication in humans by T-20, a peptide inhibitor of gp41-medicated virus entry. Nature Medicine. Vol. 3, No. 11: 1302.

## Claims

1. An anti-CCR5 chemokine receptor monoclonal antibody or a fragment thereof, wherein the antibody or fragment thereof binds to the same epitope as that to which monoclonal antibody PA14 binds, PA14 being the monoclonal antibody produced by a hybridoma cell line deposited under ATCC Accession No. HB-12610 and designated PA 14.

2. The anti-CCR5 chemokine receptor monoclonal antibody of claim 1.

3. The anti-CCR5 chemokine receptor monoclonal antibody fragment of claim 1.

4. The antibody or fragment of any of claims 1-3, wherein the antibody is humanized.

5. The antibody or fragment of any of claims 1-4 comprising complementarity determining regions (CDRs) derived from the PA14 monoclonal antibody or hybridoma.

6. The antibody of claim 4, wherein the antibody comprises a framework derived from a human immunoglobulin molecule.

7. The antibody of claim 6, wherein the human immunoglobulin molecule is selected from the group consisting of IgG1, IgG2, IgG3, IgG4, IgA and IgM.

8. The antibody of claim 7, wherein the human immunoglobulin molecule is IgG4.

9. The antibody of claim 7, wherein the human immunoglobulin molecule is IgG2.

10. The antibody or fragment of claims 1-3, wherein the antibody is a chimeric antibody.

11. The monoclonal antibody fragment of claim 3, wherein the fragment is a monovalent fragment.

12. The anti-CCR5 chemokine receptor monoclonal antibody of claim 2 designated PA14.

13. The humanized anti-CCR5 chemokine receptor monoclonal antibody of claims 4-9, wherein the antibody is a humanized antibody derived from the monoclonal antibody designated PA14.

14. The monoclonal antibody or fragment of any of claims 1-13 which specifically binds to CCR5$^+$ cells.

15. Use of the monoclonal antibody or fragment of any of claims 1-14 for the preparation of a composition for inhibiting HIV-1 fusion to, and viral entry into, CD4$^+$ cells.

## Patentansprüche

1. Monoklonaler Anti-CCR5-Chemokinrezeptor-Antikörper oder ein Fragment davon, wobei der Antikörper oder das Fragment davon an das gleiche Epitop bindet, an das der monoklonale Antikörper PA14 bindet, und PA14 der monoklonale Antikörper ist, der von einer Hybridomzelllinie produziert wird, die unter der ATCC Accession No. HB-12610 hinterlegt ist und als PA14 bezeichnet wird.

**2.** Monoklonaler Anti-CCR5-Chemokinrezeptor-Antikörper nach Anspruch 1.

**3.** Fragment des monoklonalen Anti-CCR5-Chemokinrezeptor-Antikörpers nach Anspruch 1.

**4.** Antikörper oder Fragment nach einem der Ansprüche 1 bis 3, wobei der Antikörper humanisiert ist.

**5.** Antikörper oder Fragment nach einem der Ansprüche 1 bis 4, umfassend complementarity determining regions (CDRs), die von dem monoklonalen Antikörper PA14 oder dem Hybridom abgeleitet sind.

**6.** Antikörper nach Anspruch 4, wobei der Antikörper ein Gerüst umfasst, das von einem humanen Immunglobulin abgeleitet ist.

**7.** Antikörper nach Anspruch 6, wobei das humane Immunglobulinmolekül ausgewählt ist aus der Gruppe bestehend aus IgG1, IgG2, IgG3, IgG4, IgA und IgM.

**8.** Antikörper nach Anspruch 7, wobei das humane Immunglobulinmolekül IgG4 ist.

**9.** Antikörper nach Anspruch 7, wobei das humane Immunglobulinmolekül IgG2 ist.

**10.** Antikörper oder Fragment nach den Ansprüchen 1 bis 3, wobei der Antikörper ein chimärer Antikörper ist.

**11.** Fragment des monoklonalen Antikörpers nach Anspruch 3, wobei das Fragment ein monovalentes Fragment ist.

**12.** Monoklonaler Anti-CCR5-Chemokinrezeptor-Antikörper nach Anspruch 2, bezeichnet als PA14.

**13.** Humanisierter monoklonaler Anti-CCR5-Chemokinrezeptor-Antikörper nach den Ansprüchen 4 bis 9, wobei der Antikörper ein humanisierter Antikörper ist, der von dem als PA14 bezeichneten monoklonalen Antikörper abgeleitet ist.

**14.** Monoklonaler Antikörper oder Fragment nach einem der Ansprüche 1 bis 13, der/das spezifisch an CCR5$^+$-Zellen bindet.

**15.** Verwendung des monoklonalen Antikörpers oder Fragments nach einem der Ansprüche 1 bis 14 für die Herstellung einer Zusammensetzung zum Inhibieren der HIV-1 Fusion an und des Zelleintritts des Virus in CD4$^+$-Zellen.

**Revendications**

**1.** Anticorps monoclonal anti-récepteur de chimiokines CCR5 ou un fragment de celui-ci, l'anticorps ou le fragment de celui-ci se liant au même épitope que celui auquel l'anticorps monoclonal PA14 se lie, PA14 étant l'anticorps monoclonal produit par une lignée de cellules d'hybridome déposée sous le n˚ d'accession ATCC HB-12610 et désigné PA14.

**2.** Anticorps monoclonal anti-récepteur de chimiokines CCR5 selon la revendication 1.

**3.** Fragment d'anticorps monoclonal anti-récepteur de chimiokines CCR5 selon la revendication 1.

**4.** Anticorps ou fragment selon l'une quelconque des revendications 1 à 3, l'anticorps étant humanisé.

**5.** Anticorps ou fragment selon l'une quelconque des revendications 1 à 4 comprenant des régions déterminant la complémentarité (CDR) dérivées de l'anticorps monoclonal PA14 ou d'un hybridome.

**6.** Anticorps selon la revendication 4, l'anticorps comprenant une structure dérivée d'une molécule d'immunoglobuline humaine.

**7.** Anticorps selon la revendication 6, dans lequel la molécule d'immunoglobuline humaine est choisie dans le groupe constitué par l'IgG1, l'IgG2, l'IgG3, l'IgG4, l'IgA et l'IgM.

**8.** Anticorps selon la revendication 7, dans lequel la molécule d'immunoglobuline humaine est l'IgG4.

**9.** Anticorps selon la revendication 7, dans lequel la molécule d'immunoglobuline humaine est l'IgG2.

**10.** Anticorps ou fragment selon les revendications 1 à 3, l'anticorps étant un anticorps chimérique.

**11.** Fragment d'anticorps monoclonal selon la revendication 3, le fragment étant un fragment monovalent.

**12.** Anticorps monoclonal anti-récepteur de chimiokines CCR5 selon la revendication 2 désigné PA14.

**13.** Anticorps monoclonal humanisé anti-récepteur de chimiokines CCR5 selon les revendications 4 à 9, l'anticorps étant un anticorps humanisé dérivé de l'anticorps monoclonal désigné PA14.

**14.** Anticorps monoclonal ou fragment selon l'une quelconque des revendications 1 à 13 qui se lie spécifiquement à des cellules CCR5$^+$.

**15.** Utilisation de l'anticorps monoclonal ou du fragment selon l'une quelconque des revendications 1 à 14 pour la préparation d'une composition pour inhiber la fusion du VIH-1 à, et l'entrée virale dans, des cellules CD4$^+$.

# FIGURE 1

| mAb | Mean Fluorescence Intensity | | % Gated | |
|---|---|---|---|---|
| | L1.2-CCR5 | PBMC | L1.2-CCR5 | PBMC |
| mouse IgG1 | 10 | 4 | 1 | 1 |
| 2D7 | 75 | 29 | 92 | 36 |
| PA8 | 48 | 9 | 73 | 3 |
| PA9 | 79 | 5 | 96 | 3 |
| PA10 | 80 | 8 | 96 | 5 |
| PA11 | 107 | 8 | 96 | 10 |
| PA12 | 115 | 8 | 96 | 8 |
| PA14 | 81 | 14 | 96 | 22 |

# FIGURE 2

| INHIBITOR COMBINATION | CONCENTRATION RATIO | ASSAY | COMBINATION INDEX | |
|---|---|---|---|---|
| | | | 90% Inhibition | 50% Inhibition |
| PA12:2D7 | 10:1 | Entry | 0.043 | 0.291 |
| | 2:1 | Fusion | 0.017 | 0.019 |
| | 10:1 | Fusion | 0.087 | 0.067 |
| | 50:1 | Fusion | 0.158 | 0.046 |
| PA12:PA14 | 10:1 | Entry | 0.437 | 0.535 |
| | 10:1 | Fusion | 0.22 | 0.263 |
| PA14:2D7 | 1:1 | Entry | 2.85 | 1.85 |
| | 1:1 | Fusion | 1.34 | 1.74 |
| PA12:PA11 | 1:1 | Entry | 0.707 | 0.641 |
| PA12:RANTES | 1000:1 | Fusion | 0.331 | 0.156 |
| PA14:RANTES | 100:1 | Fusion | 1.6 | 1.37 |
| 2D7:RANTES | 100:1 | Fusion | 0.972 | 0.962 |
| PA12:CD4-IgG2 | 10:1 | Fusion | 0.3 | 0.31 |
| PA14:CD4-IgG2 | 1:1 | Fusion | 0.957 | 0.566 |
| 2D7:CD4-IgG2 | 1:1 | Fusion | 1.127 | 0.302 |

# FIGURE 3

| Epitopes | IC$_{50}$ values (µg/ml) | | | |
| | cell-cell fusion inhibition | viral entry inhibition | gp120-binding inhibition | calcium flux inhibition |
| --- | --- | --- | --- | --- |
| PA8 Nt | - | - | - | - |
| PA9 Nt/ECL2 | - | - | 0.24 | - |
| PA10 Nt/ECL2 | - | - | 0.13 | - |
| PA11 Nt | 25.5 | - | 0.33 | - |
| PA12 Nt | 10.0 | - | 0.24 | - |
| PA14 Nt/ECL2 | 1.7 | .024 | 1.58 | 6.4 |
| 2D7 ECL2 | 1.6 | .026 | 1.38 | 45 |

*FIGURE 4*

76%-100+%
51%-75%
26%-50%
0%-25%
nd

EP 1 144 006 B1

# FIGURE 5A

## FIGURE 5B

# FIGURE 6A

## FIGURE 6B

FIGURE 6C

# FIGURE 6D

# FIGURE 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9930345 W

- US 09212793 B


**Non-patent literature cited in the description**

- **ALLAWAY, G.P. ; K.L. DAVIS-BRUNO ; B.A. BEAUDRY ; E.B. GARCIA ; E.L. WONG ; A.M. RYDER ; K.W. HASEL ; M.C. GAUDUIN ; R.A. KOUP ; J.S. MCDOUGAL.** Expression and characterization of CD4-IgG2, a novel heterotetramer that neutralizes primary HIV type 1 isolates. *AIDS Res Hum Retroviruses,* 1995, vol. 11, 533-539
- **ALLAWAY, G.P. ; A.M. RYDER ; G.A. BEAUDRY ; P.J. MADDON.** Synergistic inhibition of HIV-1 envelope-mediated cell fusion by CD4-based molecules in combination with antibodies to gp120 or gp41. *AIDS Res Hum Retroviruses,* 1993, vol. 9, 581-587
- **AMARA, A. ; S.L. GALL ; O. SCHWARTZ ; J. SALAMERO ; M. MONTES ; P. LOETSCHER ; M. BAGGIOLINI ; J.L. VIRELIZIER ; F. ARENZA-NA-SEISDEDOS.** HIV coreceptor downregulation as antiviral principle: SDF-1a-dependent internalization of the chemokine receptor CXCR4 contributes to inhibition of HIV replication. *J. Exp. Med.,* 1997, vol. 186, 139-146
- **BERGER, E.A.** HIV entry and tropism: the chemokine receptor connection. *AIDS,* 1997, vol. 11, S3-S16
- **BIENIASZ, P.D. ; B.R. CULLEN.** Chemokine receptors and human immunodeficiency virus infection. *Frontiers in Bioscience,* 1998, vol. 3, d44-58
- **BIENIASZ, P.D. ; R.A. FRIDELL ; I. ARAMORI ; S.S.G. FERGUSON ; M.C. CARON ; B.R. CULLEN.** HIV-1-induced cell fusion is mediated by multiple regions within both the viral envelope and the CCR5 co-receptor. *EMBO,* 1997, vol. 16, 2599-2609
- **BRELOT, A. ; N. HEVEKER ; O. PLESKOFF ; N. SOL ; M. ALIZON.** Role of the first and third extracellular domains of CXCR4 in human immunodeficiency virus coreceptor activity. *J. Virol.,* 1997, vol. 71, 4744-4751
- **CHAN, D.C. ; P.S. KIM.** HIV entry and its inhibition. *Cell,* 1998, vol. 93, 681-684
- **CHOU, T.C. ; D.C. RIDEOUT.** Synergism and antagonism in chemotherapy. Academic Press, 1991

- **COCCHI, F. ; A.L. DEVICO ; A. GARZINO-DERNO ; S.K. ARYA ; R.C. GALLO ; P.P. LUSSO.** Identification of RANTES, MIP-1α and MIP-1β as the major HIV-suppressive factors produced by CD8 T-cells. *Science,* 1995, vol. 270, 1811-1815
- **CONNOR, R.I. ; K.E. SHERIDAN ; D. CERADINI ; S. CHOE ; N.R. LANDAU.** Change in co-receptor use correlates with disease progression in HIV-1 infected individuals. *J. Exp. Med.,* 1997, vol. 185, 621-628
- **CRUMP, M.P. ; J.H. GONG ; P. LOETSCHER ; K. RAJARATHNAM ; A. AMARA ; F. ARENZANA-SEISDEDOS ; J.L. VIRELIZIER ; M. BAGGIOLINI ; B.D. SYKES ; I. CLARK-LEWIS.** Solution structure and basis for functional activity of stromal-cell derived factor-1; disassociation of CXCR4 activation from binding and inhibition of HIV-1. *EMBO,* 1997, vol. 16, 6996-7007
- **DALGLEISH, A.G. ; P.C.L. BEVERLY ; P.R. CLAPHAM ; D.H. CRAWFORD ; M.F. GREAVES ; R.A. WEISS.** The CD4 (T4) antigen is an essential component of the receptor for the AIDS retrovirus. *Nature,* 1984, vol. 312, 763-766
- **DENG, H.K. ; R. LIU ; W. ELLMEIER ; S. CHOE ; D. UNUTMAZ ; M. BURKHART ; P. DIMARIZIO ; S. MARMON ; R.E. SUTTON ; C.M. HILL.** Identification of a major co-receptor for primary isolates of HIV-1. *Nature,* 1996, vol. 381, 661-666
- **DIMITROV, D.S.** How do viruses enter cells? The HIV Co-receptors teach us a lesson of complexity. *Cell,* 1997, vol. 91, 721-730
- **DONZELLA, G.A. ; D. SCHOLS ; S.W. LIN ; K.A. NAGASHIMA ; P.J. MADDON ; G.P. ALLAWAY ; T.P. SAKMAR ; E.D. CLERCQ ; J.P. MOORE.** JM3100, a small molecule that interacts with the CXCR4 co-receptor to prevent HIV-1 entry. *Nat.,* 1998, vol. 4, 72-77
- **DORANZ, B.J. ; K. GROVIT-FERBAS ; M.P. SHARRON ; S.H. MAO ; M.B. GOETZ ; E.S. DAAR ; R.W. DOMS ; W.A. O'BRIEN.** A small molecule inhibitor directed against the chemokine receptor CXCR4 prevents its use as an HIV-1 co-receptor. *J. Ex. Med.,* 1997, vol. 186, 1395-1400

- **DORANZ, B.J. ; Z.-H. LU ; J. RUCKER ; T.-Y. ZHANG ; M. SHARRON ; Y.-H. CEN ; Z.-X. WANG ; H.-H. GUO ; J.-G. DU ; M.A. ACCAVITTI.** Two distinct CCR5 domains can mediate co-receptor usage by human immunodeficiency virus type 1. *J. Virol.,* 1997, vol. 71, 6305-6314
- **DRAGIC, T. ; V. LITWIN ; G.P. ALLAWAY ; S.R. MARTIN ; Y. HUANH ; K.A. NAGASHIMA ; C. CAYANAN ; P.J. MADDON ; R.A. KOUP ; J.P. MOORE.** HIV-1 entry into CD4+ cells is mediated by the chemokine receptor CC-CKR-5. *Nature,* 1996, vol. 381, 667-673
- **DRAGIC, T. ; A. TRKOLA ; X.W. LIN ; K.A. NAGASHIMA ; F. KAJUMO ; L. ZHAO ; W.C. OLSON ; L. WU ; C.R. MACKAY ; G.P. ALLAWAY.** Amino terminal substitutions in the CCR5 co-receptor impair gp120 binding and human immunodeficiency virus type 1 entry. *J. Virol.,* 1998, vol. 72, 279-285
- **DRAGIC, T. ; A. TRKOLA ; J.P. MOORE.** HIV co-receptors: Gateways to the cell. *Advances in Research and Therapy,* 1997, vol. 7, 2-13
- **FARZAN, M. ; H. CHOE ; L. VACA ; K. MARTIN ; Y. SUN ; E. DESJARDINS ; N. RUFFING ; L. WU ; R. WYATT ; N. GERARD.** A tyrosine-rich region in the N-terminus of CCR5 is important for human immunodeficiency virus type 1 entry and mediates an association between gp120 and CCR5. *J. Virol.,* 1998, vol. 72, 1160-1164
- **FUERST, T.R. ; E.G. NILES ; F.W. STUDIER ; B. MOSS.** Eukaryotic transient-expression system based on recombinant vaccinia virus that synthesizes bacteriophage T7 RNA polymerase. *Proc. Natl. Acad. Sci. USA.,* 1986, vol. 83, 8122-8126
- **GENOUD, S. ; F. KAJUMO ; Y. GUO ; D.A.D. THOMPSON ; T. DRAGIC.** CCR5-mediated human immunodeficiency virus entry depends on an amino-terminal domain gp120-binding site and on the conformational integrity of all four extracellular domains. *J. Virol.*
- **GETHER, U. ; B.K. KOBILKA.** G protein-coupled receptors. *J. Biol. Chem,* 1998, vol. 273, 17979-17982
- **GORDON, C. ; M. MUESING ; A.E.I. PROUDFOOT ; C.A. POWER ; J.P. MOORE ; A. TRKOLA.** Enhancement of human immunodeficiency virus type 1 infection by the CC-chemokine RANTES is independent of the mechanism of virus-cell fusion. *J. Virol.,* 1998
- **HEVEKER, N. ; M. MONTES ; L. GERMEROTH ; A. AMARA ; A. TRAUTMANN ; M. ALIZON ; J. SCHNEIDER-MERGENER.** Dissociation of the signaling and antiviral properties of SDF-1-derived small peptides. *Current Biology,* 1998, vol. 8, 369-376
- **HILL, C.M. ; D. KWON ; M. JONES ; C.B. DAVIS ; S. MARMON ; B.L. DAUGHERTY ; J.A. DEMARTINO ; M.S. SPRINGER ; D. UNUTMAZ ; D.R. LITTMAN.** The amino terminus of human CCR5 is required for its function as a receptor for diverse human and simian immunodeficiency virus envelope glycoproteins. *Virology,* 1998, vol. 248, 357-371
- **JOHNSON, V.A. ; D.P. MERRILL ; J.A. VIDELER ; T.C. CHOU ; R.E. BYINGTON ; J.J. ERON ; R.T. D'AQUILA ; M.S. HIRSCH.** Two-drug combinations of zidovudine, didanosine, and recombinant interferon-alpha A inhibit replication of zidovudine-resistant human immunodeficiency virus type 1 synergistically in vitro. *J Infect Dis,* 1991, vol. 164, 646-655
- **KLATZMANN, D. ; E. CHAMPAGNE ; S. CHAMARET ; J.M. GRUEST ; D. GUETARD ; T. HERCEND ; J.C. GLUCKMAN ; L. MONTAGNIER.** T-lymphocyte T4 molecule behaves as the receptor for human retrovirus LAV. *Nature,* 1984, vol. 312, 382-385
- **KUHMANN, K.E. ; E.J. PLATT ; S.L. KOZAK ; D. KABAT.** Polymorphism in the CCR5 genes of African green monkeys and mice implicate specific amino acids in infections by simian and human immunodeficiency viruses. *J. Virol.,* 1997, vol. 71, 8642-8656
- **KWONG, P.D. ; R. WYATT ; J. ROBINSON ; R.W. SWEET ; J. SODROSKI ; W.A. HENDRICKSON.** Structure of an HIV gp120 envelope glycoprotein in complex with the CD4 receptor and a neutralizing human antibody. *Nature,* 1998, vol. 393, 648-659
- **LAAL, S. ; S. BURDA ; M.K. GORNY ; S. KARWOWSKA ; A. BUCHBINDER ; S. ZOLLA-PAZNER.** Synergistic neutralization of human immunodeficiency virus type 1 by combinations of human monoclonal antibodies. *J. Virol.,* 1994, vol. 68, 4001-4008
- **LABROSSE, B. ; A. BRELOT ; N. HEVEKER ; N. SOL ; D. SCHOLS ; E.D. CLERCQ ; M. ALIZON.** Determinants for sensitivity of human immunodeficiency virus co-receptor CXCR4 to the bicyclam AMD3100. *J. Virol.,* 1998, vol. 72, 6381-6388
- **LI, A. ; H. KATINGER ; M.R. POSNER ; L. CAVACINI ; S. ZOLLA-PAZNER ; M.K. GORNY ; J. SODROSKI ; T.C. CHOU ; T.W. BABA ; R.M. RUPRECHT.** Synergistic neutralization of simian-human immunodeficiency virus SHIV-vpu+ by triple and quadruple combinations of human monoclonal antibodies and high-titer anti-human immunodeficiency virus type 1 immunoglobulins. *J. Virol.,* 1998, vol. 72, 3235-3240
- **LITTMAN, D.R.** Chemokine receptors: keys to AIDS pathogenesis. *Cell,* 1998, vol. 93, 677-680

- **LITWIN, V. ; K. NAGASHIMA ; A.M. RYDER ; C.H. CHANG ; J.M. CARVER ; W.C. OLSON ; M. ALIZON ; K.W. HASEL ; P.J. MADDON ; G.P. AL-LAWAY.** Human immunodeficiency virus type 1 membrane fusion mediated by a laboratory-adapted strain and a primary isolate analyzed by resonance energy transfer. *J. Virol.,* 1996, vol. 70, 6437-6441
- **LOETSCHER, P. ; J.H. GONG ; B. DEWALD ; M. BAGGIOLONI ; I. CLARK-LEWIS.** N-terminal peptides of stromal cell derived factor-1 with CXC chemokine receptor 4 agonist and antagonist activities. *J. Biol. Chem.,* 1998, vol. 273, 22279-22283
- **MACK, M. ; B. LUCKOW ; P.J. NELSON ; J. CIHAK ; G. SIMMONS ; P.R. CLAPHAM ; N. SIGNORET ; M. MARSH ; M. STANGASSINGER ; F. BORLAT.** Aminooxypentane-RANTES induces CCR5 internalization but inhibits recycling: a novel inhibitory mechanisms of HIV infectivity. *J. Ex. Med.,* 1998, vol. 187, 1215-1224
- **MADDON, P.J. ; A.G. DALGLEISH ; J.S. MCDOUGAL ; P.R. CLAPHAM ; R.A. WEISS ; R. AXEL.** The T4 gene encodes the AIDS virus receptor and is expressed in the immune system and the brain. *Cell,* 1986, vol. 47, 333-348
- **MCDOUGAL, J.S. ; M.S. KENNEDY ; J.M. SLIGH ; S.P. CORT ; A. MAWLE ; J.K.A. NICHOLSON.** Binding of HTLVIII / LAV to T4+ T cells by a complex of the 110K viral protein and the T4 molecule. *Science,* 1986, vol. 231, 382-385
- **MCKNIGHT, A. ; D. WILKINSON ; G. SIMMONS ; S. TALBOT ; L. PICARD ; M. AHUJA ; M. MARSH ; J.A. HOXIE ; P.R. CLAPHAM.** Inhibition of human immunodeficiency virus fusion by a monoclonal antibody to a co-receptor (CXCR4) is both cell type and virus strain dependent. *J. Virol.,* 1997, vol. 71, 1692-1696
- **MERRILL, D.P. ; D.J. MANION ; T.C. CHOU ; M.S. HIRSCH.** Antagonism between human immunodeficiency virus type 1 protease inhibitors indinavir and saquinavir in vitro. *J Infect Dis,* 1997, vol. 176, 265-268
- **MOORE, J.P. ; Y. CAO ; L. QING ; Q.J. SATTENTAU ; J. PYATI ; R. KODURI ; J. ROBINSON ; C.F. BARBAS ; D.R. BURTON ; D.D. HO.** Primary isolates of human immunodeficiency virus type 1 are relatively resistant to neutralization by monoclonal antibodies to gp120 and their neutralization is not predicted by studies with monomeric gp120. *J. Virol.,* 1995, vol. 69, 101-109
- **MOORE, J.P. ; B.A. JAMESON ; R.A. WEISS ; Q.J. SATTENTAU.** The HIV-cell fusion reaction. CRC Press Inc, 1993
- **MOORE, J.P. ; J.A. MCKEATING ; Y. HUANG ; A. ASHKENAZI ; D.D. HO.** Virions of primary human immunodeficiency virus type 1 isolates resistant to soluble CD4 (sCD4) neutralization differ in sCD4 binding and glycoprotein gp120 retention from sCD4-sensitive isolates. *J. Virol.,* 1992, vol. 66, 235-243
- **MOORE, J.P. ; R.W. SWEET.** The HIV gp120-CD4 interaction: a target for pharmacological and immunological intervention. *Prospect in Drug Discovery and Design,* 1993, vol. 1, 235-250
- **MURAKAMI, T. ; T. NAKAJIMA ; Y. KOYANAGI ; K. TACHIBANA ; N. FUJII ; H. TAMAMURA ; N. YOSHIDA ; M. WAKI ; A. MATSUMOTO ; O. YOSHIE.** A small molecule CXCR4 inhibitor that blocks T cell line-tropic HIV-1 infection. *J. Ex. Med.,* 1997, vol. 186, 1389-1393
- **PANTALEO, G. ; G. POLI ; L. BUTINI ; C. FOX ; A.I. DAYTON ; A.S. FAUCI.** Dissociation between syncytia formation and HIV spreading. Suppression of syncytia does not necessarily reflect inhibition of HIV infection. *Eur. J. Immunol.,* 1991, vol. 21, 1771-1774
- **RABUT, G.E.E. ; J.A. KONNER ; F. KAJUMO ; J.P. MOORE ; T. DRAGIC.** Alanine substitutions of polar and non-polar residues in the amino-terminal domain of CCR5 differently impair entry of macrophage- and dual-tropic isolates of the human immunodeficiency virus type 1. *J. Virol.,* 1998, vol. 72, 3464-3468
- **RIZZUTO, C. ; R. WYATT ; N. HERNANDEZ-RAMOS ; Y. SUN ; P. KWONG ; W. HENDRICKSON ; J. SODROSKI.** Identification of a conserved human immunodeficiency virus gp120 glycoprotein structure important for chemokine receptor binding. *Science,* 1998, vol. 280, 1949-1953
- **RUCKER, J. ; M. SAMSON ; B.J. DORANZ ; F. LIBERT ; J.F. BERSON ; Y. YI ; R.J. SMYTH ; R.G. COLLMAN ; C.C. BRODER ; G. VASSART.** Regions in the β-chemokine receptors CCR-5 and CCR-2b that determine HIV-1 cofactor specificity. *Cell,* 1996, vol. 87, 437-446
- **SCHOLS, D. ; S. STRUYF ; J.V. DAMME ; J.A. ESTE ; G. HENSON ; E.D. CLERCQ.** Inhibition of T-tropic HIV strains by selective antagonization of the chemokine receptor CXCR4. *J. Ex. Med.,* 1997, vol. 186, 1383-1388
- **SIMMONS, G. ; P.R. CLAPHAM ; L. PICARD ; R.E. OFFORD ; M.M. ROSENKILDE ; T.W. SCHWARTZ ; R. BUSER ; T.N.C. WELLS ; A.E.I. PROUDFOOT.** Potent inhibition of HIV-1 infectivity in macrophages and lymphocytes by a novel CCR5 antagonist. *Science,* 1997, vol. 276, 276-279

- **SIMMONS, G. ; D. WILKINSON ; J.D. REEVES ; M.T. DITTMAR ; S. BEDDOWS ; J. WEBER ; G. CARNEGIE ; U. DESSELBERGER ; P.W. GRAY ; R.A. WEISS.** Primary, syncytium-inducing human immunodeficiency virus type-1 isolates are dual-tropic and most can use either LESTR or CCR5 as co-receptor for virus entry. *J. Virol.,* 1996, vol. 70, 8355-8360
- **STRIZKI, J.M. ; J. DAVIS-TURNER ; R.G. COLLMAN ; J. HOXIE ; F. GONZALEZ-SCARANO.** A monoclonal antibody (12G5) directed against CXCR4 inhibits infection with the dual-tropic human immunodeficiency virus type 1 isolate HIV-1 89.6 but not the T-tropic isolate HIV-1 HxB. *J. Virol.,* 1997, vol. 71, 5678-5683
- **TRKOLA, A. ; T. DRAGIC ; J. ARTHOS ; J. BINLEY ; W.C. OLSON ; G.P. ALLAWAY ; C. CHENG-MAYER ; J. ROBINSON ; P.J. MADDON ; J.P. MOORE.** CD4-dependent, antibody sensitive interactions between HIV-1 and its co-receptor CCR-5. *Nature,* 1996, vol. 384, 184-187
- **TRKOLA, A. ; W.A. PAXTON ; S.P. MONARD ; J.A. HOXIE ; M.A. SIANI ; D.A. THOMPSON ; L. WU ; C.R. MACKAY ; R. HORUK ; J.P. MOORE.** Genetic subtype-independent inhibition of human immunodeficiency virus type-1 replication by CC- and CXC chemokines. *J. Virol.,* 1997, vol. 72, 396-404
- **VIJH-WARRIER, S. ; A. PINTER ; W.J. HONNEN ; S.A. TILLEY.** Synergistic neutralization of human immunodeficiency virus type 1 by a chimpanzee monoclonal antibody against the V2 domain of gp120 in combination with monoclonal antibodies against the V3 loop and the CD4-binding site. *J. Virol.,* 1996, vol. 70, 4466-4473
- **WARD, S.G. ; K. BACON ; J. WESTWICK.** Chemokines and lymphocytes: more than an attraction. *Immunity,* 1998, vol. 9, 1-11
- **WU, L. ; N.P. GERARD ; R. WYATT ; H. CHOE ; C. PAROLIN ; N. RUFFING ; A. BORSETTI ; A.A. CARDOSO ; E. DESJARDIN ; W. NEWMAN.** CD4-induced interaction of primary HIV-1 gp120 glycoproteins with the chemokine receptor CCR-5. *Nature,* 1996, vol. 384, 179-183
- **WU, L. ; G. LAROSA ; N. KASSAM ; C.J. GORDON ; H. HEATH ; N. RUFFING ; H. CHEN ; J. HUMBLIAS ; M. SAMSON ; M. PARMENTIER.** Interaction of chemokine receptor CCR5 with its ligands: multiple domains for HIV-1 gp120 binding and a single domain for chemokine binding. *J. Exp. Med.,* 1997, vol. 186, 1373-1381
- **WYATT, R. ; P.D. KWONG ; E. DESJARDINS ; R. SWEET ; J. ROBINSON ; W. HENDRICKSON ; J. SODROSKI.** The antigenic structure of the human immunodeficiency virus gp120 envelope glycoprotein. *Nature,* 1998, vol. 393, 705-711
- **WYATT, R. ; J. SODROSKI.** The HIV-1 envelope glycoproteins: fusogens, antigens and immunogens. *Science,* 1998, vol. 280, 1884-1888
- **YLISASTIGUI, L. ; J.J. VIZZAVONA ; E. DRAKOPOULOU ; P. PAINDAVOINE ; C.F. CALVO ; M. PARMENTIER ; J.C. GLUCKMAN ; C. VITA ; A. BENJOUAD.** Synthetic full length and truncated RANTES inhibit HIV-1 infection of primary macrophages. *AIDS,* 1998, vol. 12, 977-984
- **ZHANG, J.L. ; H. CHOE ; B.J. DEZUBE ; M. FARZAN ; P.L. SHARMA ; X.C. ZHOU ; L.B. CHEN ; M. ONO ; S. GILLIES ; Y. WU.** The bis-azo compound FP-21399 inhibits HIV-1 replication by preventing viral entry. *Virology,* 1998, vol. 244, 530-541
- **CAIRNS, J.S. ; D'SOUZA. M.P.** Chemokines and HIV-1 second receptors: the therapeutic connection. *Nature Medicine.,* 1998, vol. 4 (5), 563
- **KILBY ; J.MICHAEL et al.** Potent suppression of HIV-1 replication in humans by T-20, a peptide inhibitor of gp41-medicated virus entry. *Nature Medicine.,* 1998, vol. 3 (11), 1302

SEQUENCE LISTING

<110> Progenics Pharmaceuticals, Inc.
OLSON, WILLIAM C.
MADDON, PAUL J.

<120> SYNERGISTIC INHIBITION OF OF HIV-1 FUSION AND ATTACHMENT, COMPOSITIONS AND ANTIBODIES THERETO

19

<130> 57906-PCT-EPO

<140> PCT/US99/30345
<141> 1999-12-16

<150> US 09/212,793
<151> 1998-12-16

<150> us 60/112,532
<151> 1998-12-16

<160> 4

<170> PatentIn version 3.1

<210> 1
<211> 31
<212> PRT
<213> HUMAN

<400> 1

```
Met Asp Tyr Gln Val Ser Ser Pro Ile Tyr Asp Ile Asn Tyr Tyr Thr
1               5                   10                  15

Ser Glu Pro Cys Gln Lys Ile Asn Lys Gln Ile Ala Ala Ala Arg
            20              25              30
```

<210> 2
<211> 15
<212> PRT
<213> HUMAN

<400> 2

```
His Tyr Ala Ala Ala Gln Trp Asp Phe Gly Asn Thr Met Cys Gln
1               5                   10                  15
```

<210> 3
<211> 27
<212> PRT
<213> HUMAN

<400> 3 ,

```
Arg Ser Gln Lys Glu Gly Leu His Tyr Thr Cys Ser Ser His Phe Pro
1               5                   10                  15

Tyr Ser Gln Tyr Gln Phe Trp Lys Asn Phe Gln
            20              25
```

<210> 4
<211> 17

<212> PRT
<213> HUMAN

<400> 4

```
Gln Glu Phe Phe Gly Leu Asn Asn Cys Ser Ser Ser Asn Arg Leu Asp
1               5               10                      15

Gln
```